# EUROPEAN PATENT SPECIFICATION

(11) **EP 3 187 191 B1**
(45) Date of publication and mention of the grant of the patent: **08.04.2020**
(21) Application number: 15836263.2
(22) Date of filing: 26.08.2015
(51) Int. Cl.: A61K 39/395, A61K 38/17, A61K 38/16, A61P 35/00, G01N 33/50

(54) **ANTIBODY FOR RECOGNIZING SPECIFIC MOTIF OF WLS PROTEIN, AND PHARMACEUTICAL COMPOSITION CONTAINING SAME**
ANTIKÖRPER ZUR ERKENNUNG EINES SPEZIFISCHEN MOTIVS EINES WLS-PROTEINS UND PHARMAZEUTISCHE ZUSAMMENSETZUNG DAMIT
ANTICORPS DE RECONNAISSANCE DE MOTIF SPÉCIFIQUE DE PROTÉINE WLS, ET COMPOSITION PHARMACEUTIQUE LE CONTENANT

(30) Priority: 26.08.2014 KR 20140111398
(43) Date of publication of application: 05.07.2017
(73) Proprietor: NKMAX Co., Ltd., South Korea (KR)
(72) Inventor: YOON, Ho Geun, Goyang-si Gyeonggi-do 10529 (KR); CHEONG, Jae Ho, Seoul 103-808 (KR); PARK, Eun Sung, Seoul 06522 (KR); SEO, Jae Sung, Seoul 03907 (KR); KEE, Hyun Jung, Seoul 05504 (KR)
(74) Representative: Michalski Hüttermann & Partner Patentanwälte mbB
(86) International application number: PCT/KR2015/008929
(87) International publication number: WO 2016/032230

(56) References cited:
- WO-A1-2010/014948
- WO-A2-2004/024077
- WO-A2-2006/097336
- WO-A2-2012/143382
- KR-A- 20080 040 738
- HANFENG XU ET AL: "Expression of Wntless in colorectal carcinomas is associated with invasion, metastasis, and poor survival", APMIS, vol. 124, no. 6, 22 April 2016 (2016-04-22), pages 522-528, XP55308035, DK ISSN: 0903-4641, DOI: 10.1111/apm.12534
- AUGUSTIN, IRIS ET AL.: 'The Wnt secretion protein Evi/Gpr177 promotes glioma tumourigenesis' EMBO MOLECULAR MEDICINE vol. 4, no. 1, 01 January 2011, pages 38 - 51, XP002678954 DOI: 10.1002/EMMM.201100186
- STEWART, JONATHAN ET AL.: 'A strong correlation between expression of Wntless and of human epidermal growth factor receptor 2 in gastric, ovarian, and breast cancers suggests a novel-signalling pathway involving NFkappaB and STAT3' THE LANCET vol. 381, no. 1, 27 February 2013, page 106, XP055411963

## Description

### [Technical Field]

The present invention relates to an antibody that recognizes a specific motif of WLS protein so as to inhibit overactivation of the Wnt signaling pathway to thereby prevent or treat a disease associated with the Wnt signaling pathway, and to a pharmaceutical composition containing the same.

### [Background Art]

Cell signaling is a several-step process in which exogenous stimuli (hormones, growth factors, neurotransmitters, etc.) are delivered into cells to cause a variety of cellular responses, including gene expression, cell differentiation, division, growth and death. One or more impairments and abnormalities of cell signaling often cause diseases. Because four main diseases (cancer, cardiovascular disease, immune disease and brain disease) of modern people are almost all caused by abnormalities of cell signaling pathways, either discovery of drugs that target signaling proteins or screening of drugs that regulate the activities of cell signaling pathways becomes the main target of new drug development, and such drugs accounts for 50% or more of the pharmaceutical industry.

Among such cell signaling pathways, the Wnt/β-catenin signaling pathway is a signaling pathway that plays an essential role in vertebral animal development, growth and homeostatic maintenance. Wnt signaling is important in both embryonic development and homeostasis in adult animals. The Wnt pathway generally consists of a network of proteins that regulate the following processes: 1. the production and secretion of Wnt protein; 2. the binding of Wnt to cell receptors; and 3. the intracellular transduction of biochemical responses triggered by the interaction (Mikels and Nusse, 2006; MacDonald, 2009; Moon, 2005).

The so-called canonical Wnt pathway that is triggered by the binding of Wnt protein to the cell surface co-receptor Frizzled LRP5/6 results in a change in the amount of β-catenin that reaches the nucleus where it interacts with TCF/LEF family transcription factors to promote transcription of specific genes.

The non-canonical Wnt pathway transduced by a different set of intracellular proteins controls planar cell polarity in insects and several processes such as gastrulation in vertebrates.

Wnt signaling is also known for its roles in controlling pluripotency and differentiation of embryonic and adult stem cells (Nusse, 2008). For example, formation of the primitive streak during gastrulation was associated with localized Wnt activation in the embryoid bodies (ten Berge, 2008). The derivation of a number of cell types, such as heart cells, pancreatic beta cells, dopminergic neurons and liver hepatocytes from embryonic stem cells or iPS cells is influenced by Wnt modulation (Yang, 2008; D'Amour, 2006; Inestrosa and Arenas, 2010; Sullivan, 2010). The Wnt pathway plays a particularly important role in skeletal tissue development such as osteogenesis and chondrogenesis (Hoeppner, 2009; Chun, 2008). Wnt signaling is also associated with neuro-regeneration of the adult central nervous system (Lie, 2005).

Diseases may arise from altered Wnt pathway activity. For example, hyperactivation of the canonical Wnt pathway can lead to aberrant cell growth (Reya and Clevers, 2005). Notably, 90% of colorectal cancers are initiated by the loss of the adenomatosis polyposis coli (APC) gene, a suppressor of the Wnt/β-catenin pathway (Kinzler and Vogelstein, 1996). Increased expression of Wnt proteins and loss of extracellular inhibitors that normally suppress Wnt protein function may give rise to Wnt-dependent tumors (Polakis, 2007). On the other hand, the non-canonical Wnt pathway has also been shown to play a role in the progression of certain cancers (Camilli and Weeraratna, 2010). More recently, Wnt signaling is also implicated in cancer stem cells (Takahashi-Yanaga and Kahn, 2010).

Evidence suggests that targeting the Wnt-mediated signal transduction pathway would be therapeutically useful in a broad range of diseases (Barker and Clevers, 2006). Mutations of APC, beta-catenin or axin-1 leading to constitutive activation of the canonical Wnt pathway are critical events in a variety of human cancers including colorectal cancer, melanoma, hepatocellular carcinoma, gastric cancer, ovarian cancer and others (Polakis, 2007). Blockade of the Wnt pathway in a variety of cancers using either genetic or chemical approaches has been shown to abrogate aberrant cell growth (Herbst and Kolligs, 2007). Furthermore, inhibition of this pathway may directly influence the cells that sustain cancer cell growth and enable metastasis and that are resistant to traditional chemotherapeutic agents.

In addition to activation caused by mutations of gene products downstream of the receptors, aberrant Wnt pathway activity caused by other mechanisms have been associated with a broad range of cancers. These cancers include but not limited to: lung (small cell and non-small cell), breast, prostate, carcinoid, bladder, scarcinoma, esophageal, ovarian, cervical, endometrial, mesothelioma, melanoma, sarcoma, osteosarcoma, liposarcoma, thyroid, desmoids, acute myelocytic leukemia (AML), and chronic myelocytic leukemia (CML). There are now multiple examples of cancer cells dependent upon upregulated autocrine or paracrine Wnt signaling, and cell lines from osteosarcoma, breast, head and neck and ovarian cancers have been shown to derive protection from apoptosis by autocrine or paracrine Wnt signaling (Kansara, 2009; Bafico, 2004; Akiri, 2009; DeAlmeida, 2007; Chan, 2007; Chen, 2009; Rhee, 2002).

Furthermore, aberrant Wnt pathway has been implicated in the development of fibrosis, include but are not limited to: lung fibrosis, such as idiopathic pulmonary fibrosis and radiation-induced fibrosis, renal fibrosis and liver fibrosis (Morrisey, 2003; Hwang, 2009; Cheng, 2008).

Other disorders associated with aberrant Wnt signaling, include but are not limited to bone and cartilage disorders, such as osteoporosis and osteoarthritis, obesity associated type II diabetes, and neurodegenerative diseases such as Alzheimer's disease (Hoeppner, 2009; Ouchi, 2010; Blom, 2010; Boonen, 2009). Wnt signaling also contributes to the self-renewal and maintenance of HSC's, and dysfunctional Wnt signaling is responsible for various disorders resulting from HSC's, such as leukemias and various other blood related cancers (Reya, 2005).

International patent application WO 2012/143382 discloses modulators of G protein-coupled receptor 177 for use in the treatment, alleviation, prevention and/or diagnosis of aberrant Wnt signaling, in particular cancer.

### [Disclosure]

### [Technical Problem]

It is an object of the present invention to provide a pharmaceutical composition for preventing or treating a Wnt signaling pathway-associated disease, which inhibits overactivation of the Wnt signaling pathway by use of an antibody that recognizes a specific motif of the WLS protein that regulates Wnt protein secretion in Wnt signaling.

However, the technical object to be achieved by the present invention is not limited to the above technical object, and other objects that are not mentioned above can be clearly understood by those skilled in the art from the following description.

### [Technical Solution]

The present invention is defined by the composition for use of claims 1 and 3, as well as the screening method of claim 5.

Hereinafter, various embodiments and examples described herein will be described with reference to figures. In the following description, numerous specific details are set forth, such as specific configurations, compositions, and processes, etc., in order to provide a thorough understanding of the present invention. However, certain embodiments may be practiced without one or more of these specific details, or in combination with other known methods and configurations. In other instances, known processes and preparation techniques have not been described in particular detail in order to not unnecessarily obscure the present invention. Reference throughout this specification to "one embodiment" or "an embodiment" means that a particular feature, configuration, composition, or characteristic described in connection with the embodiment is included in at least one embodiment of the present invention. Thus, the appearances of the phrase "in one embodiment" or "an embodiment" in various places throughout this specification are not necessarily referring to the same embodiment of the present invention. Additionally, the particular features, configurations, compositions, or characteristics may be combined in any suitable manner in one or more embodiments.

Unless otherwise specified in the specification, all the scientific and technical terms used in the specification have the same meanings as commonly understood by those skilled in the technical field to which the present invention pertains.

The Wnt protein may be a signaling protein that is involved in a variety of developmental processes such as stem cell control in the embryogenesis phase. The Wnt protein has a molecular weight of about 40 kDa and contains several conserved cysteines. In the year 2003, mouse Wnt3a protein was first successfully purified, and was found to be a lipid-regulated protein. Lipid added to Wnt is required for effective signaling, because the lipid portion of one of regions in the Wnt protein, which bind to a receptor, spreads into a pocket-shaped receptor and binds to the receptor. Most mammals, including humans, have 19 Wnt genes, 12 of which belong to a conserved subfamily. Unicellular organisms have no Wnt gene, suggesting that Wnt signaling would play a very important role in the beginning of the evolution of multicellular animals.

WLS (Wntless) protein is a protein necessary for secretion of Wnt protein in Wnt signaling, and is also known as G protein-coupled receptor 177 (GPR177), Clorfl39, EVI, MRP, sprinter or mig-14. The WLS gene encodes a receptor for the Wnt protein in cells that secrete the Wnt protein. The WLS protein (Wnt receptor) is a heterodimeric structure composed of Frizzled protein (Fz) and lipoprotein receptor-related proteins 5/6 (LRP5/6). The Fz protein is a receptor that passes through the cell membrane seven times, and it contains an N-terminal cysteine-rich domain (CRD) and interacts with Wnt at various positions through the CRD. Fz binds to Wnt together with a receptor, known as the LRP family, which passes through the cell membrane once. The structure of the Wnt receptor is changed by ligand binding thereto, whereby major target proteins are phosphorylated. The key step in signaling is the binding of Axin to the cytoplasmic end of LRP, which is regulated by phosphorylation of the LRP6 tail. In other signaling pathways, one kinase functions to amplify signals by phosphorylating several substrates. However, LRP6 phosphorylation by Wnt functions to dilute Axin that can act as a negative regulator in a destruction complex. Namely, there is a major difference in that a stoichiometric mechanism rather than a catalytic mechanism is used.

In one embodiment of the present invention, "angiogenesis" means a cellular process in which vascular endothelial cells proliferate and are reconstituted to form new blood vessels from the existing vascular network. Because new blood vessels through which supply oxygen and nutrients are supplied are required for the proliferation and growth of cancer cells, inhibition of cancer metastasis can be induced by inhibition of angiogenesis.

In one embodiment of the present invention, "metastasis" means that cancer cells spread from their original organ to other organs. Spreading of cancer to other parts of the body is largely divided into a process in which cancer cells grow in the primary cancer and spread directly to the surrounding organs, and a process in which cancer cells metastasize to distinct organs through blood vessels or lymphatic vessels. Metastasis can be controlled by inhibiting expression of carcinogenesis-related genes or inhibiting the activity of proteins encoded by the genes.

In one embodiment of the present invention, inhibition of gene expression may be achieved by an antisense oligonucleotide, siRNA, shRNA or micro-RNA, which is complementary to a target gene.

The antisense oligonucleotide is a short synthetic DNA strand (or DNA analog) which is antisense (or complementary) to a certain DNA or RNA target. The antisense oligonucleotide is used to achieve gene- specific inhibition both *in vivo* and *in vitro.* The antisense oligonucleotide was proposed to prevent expression of the protein encoded by a DNA or RNA target. For this purpose, the antisense oligonucleotide binds to the target to thereby halt the protein expression at a transcription, translation or splicing stage. Further, the antisense oligonucleotide has been successfully used in cell culture and animal models of disease. Additional modification of antisense oligonucleotides to ensure that oligonucleotide is more stable and resistant against *in vivo* nuclease degradation may be carried out by any conventional method known to those skilled in the art. As used herein, the term "antisense oligonucleotide" encompasses double-stranded DNAs (dsDNAs) or single-stranded DNAs (ssDNAs), dsRNAs or ssRNAs, DNA/RNA hybrids, DNA and RNA analogs, and oligonucleotides having modifications of base, sugar, and/or backbone. The oligonucleotide may be chemically modified to improve the stability thereof and increase the resistance against nuclease degradation, according to a conventional method known in the art. These modification methods are conventionally known in the art, and may include, but are not limited to, modifications of the oligonucleotide backbone, sugar moieties and bases.

The siRNA (small interfering RNA) is a nucleic acid molecule that can mediate RNA interference or gene silencing. Since siRNA can inhibit the expression of a target gene, it is used as an efficient gene knockdown method or gene therapeutic method. If an siRNA molecule is used according to the present disclosure, it may have either a double-stranded structure in which a sense strand (a sequence corresponding to the WLS mRNA sequence) and an antisense strand (a sequence complementary to the WLS mRNA sequence) are located opposite to each other, or a single-stranded structure having self-complementary sense and antisense strands. The double-stranded RNA portions of siRNAs in which two RNA strands pair up are not limited to the completely paired ones, and may contain nonpairing portions due to mismatch (the corresponding nucleotides are not complementary) or bulge (lacking in the corresponding complementary nucleotide on one strand). The terminal structure of siRNA may be either blunt or cohesive as long as siRNA can inhibit the target gene expression due to its RNA interference (RNAi) effect. The cohesive end structure is not limited only to the 3' overhang, and the 5' overhanging structure may be included. The siRNA molecule has a total length of 15-30 nucleotides, preferably 19-21 nucleotides, but is not limited thereto.

The shRNA (short hairpin RNA) is a single-stranded RNA having a length of 45-70 nucleotides. When an oligo DNA that connects a 3-10-nucleotide linker between antisense strands complementary to the sense strand of the target gene siRNA sequence is synthesized and then cloned into a plasmid vector, or when shRNA is inserted and expressed in retrovirus, lentivirus or adenovirus, a looped hairpin shRNA is made and converted to siRNA by an intracellular dicer to exhibit the RNAi effect.

The microRNA regulates various biological processes, including development, differentiation, proliferation, conservation and apoptosis. The microRNA generally makes a target mRNA unstable or interferes with translation to thereby regulate expression of the gene encoding the target mRNA.

A control sequence (for example, constitutive promoter, inducible promoter, tissue-specific promoter, or a combination thereof) useful for an expression construct/vector having the antisense oligonucleotide siRNA, shRNA or microRNA) may also be suitably selected from those known in the art. In addition to the antisense oligonucleotide siRNA, shRNA or microRNA, any substance may be used as the inhibitor of expression of the gene, as long as it inhibits expression of the gene.

According to the present invention, inhibition of protein expression may be achieved by a target protein-specific antibody or an antigen-binding fragment thereof.

As used herein, the term "antibody" is a term known in the art and refers to a specific protein molecule that is directed against an antigenic site. For the purpose of the present invention, the antibody means an antibody that binds specifically to a protein expressed from the WLS gene which is the marker of the present invention. This antibody may be produced by cloning each gene into an expression vector according to a conventional method to obtain a protein encoded by the marker gene and producing the antibody from the obtained protein according to a conventional method. The proteins include a partial peptide that may be produced from the protein, and the partial peptide of the present disclosure comprises at least 7 amino acids, preferably at least 9 amino acids, more preferably at least 12 amino acids. The form of antibody according to the present disclosure is not specifically limited, and polyclonal antibodies, monoclonal antibodies, or portions thereof which have antigen binding ability are included in the antibodies of the present disclosure, and all immunoglobulin antibodies are included in the antibodies disclosed herein. Furthermore, the antibodies of the present invention also include special antibodies such as humanized antibodies. The antibodies that are used for detection of the cancer marker according to the present invention include not only a complete antibody having two full-length light chains and two full-length heavy chains, but also a functional fragment of the antibody molecule. The expression "functional fragment of the antibody molecule" refers to a fragment having at least antigen binding ability, and examples of the functional fragment include Fab, F(ab'), F(ab') 2 and Fv.

"Diagnosis" may mean identifying the presence or features of pathologic states. With respect of the object of the present invention, "diagnosis" means determination of the development and metastasis of gastric cancer.

In one embodiment, the term "marker for diagnosis", marker for diagnosing" or "diagnostic marker" means a substance capable of diagnosing cancer cells by distinguishing between normal cells and cancer cells, and includes organic biomolecules, such as polypeptides, nucleic acids (e.g., mRNA, etc.), lipids, glycolipids, glycoproteins or sugars (monosaccharides, disaccharides, oligosaccharides, etc.), the quantities of which increase or decrease in cancer cells as compared with normal cells. With respect to the object of the present invention, the marker for diagnosing cancer according to the present invention is the mRNA of WLS gene or a protein encoded thereby, which shows a higher level of expression specifically in gastric cancer cells than in the cells of normal subject tissue.

"Measurement of mRNA expression levels" may refer to a process that determines the presence and expression levels of mRNA of cancer marker genes in a biological sample in order to diagnose cancer, and the measurement of mRNA expression levels can be performed by measuring the amount of mRNA. Assays for this measurement include, but are not limited to, reverse transcription polymerase chain reaction (RT-PCR), competitive RT-PCR, real-time RT-PCR, RNase protection assay (RPA), northern blotting or DNA chip assay.

"An agent for measuring the mRNA expression level of the gene" refers to a molecule that may be used to detect the marker by determining the expression level of the WLS gene whose expression increases in cancer cells. The agent may preferably be an agent comprising a primer or probe binding specifically to the gene, but is not limited thereto. Based on the polynucleotide sequence of WLS, those skilled in the art can design a primer or probe for specifically amplifying a specific region of the gene.

As used herein, the term "primer" refers to a nucleic acid sequence having a short free 3'-end hydroxyl group, which is a short nucleic acid sequence that may form a base pair with a complementary template and act as a start point for template strand replication. The primer may initiate DNA synthesis in the presence of a reagent (e.g., DNA polymerase or reverse transcriptase) for polymerization and four different nucleoside triphosphates in suitable buffer at a suitable temperature. According to the present disclosure, PCR amplification may be performed using the sense and antisense primers of the WLS polynucleotide, and cancer can be diagnosed based on whether or not a desired product is produced by the PCR. PCR conditions and the lengths of the sense and antisense primers may be suitably selected according to techniques known in the art.

As used herein, the term "probe" refers to a fragment of nucleic acid such as RNA or DNA, which can bind specifically to mRNA and is several nucleotides to several hundred nucleotides in length. The probe can determine the presence or absence of a specific mRNA, because it is labeled. The probe can be constructed as an oligonucleotide probe, a single-stranded DNA probe, a double-stranded DNA probe, an RNA probe or the like. In the present invention, cancer can be diagnosed by performing hybridization using a probe complementary to the WLS polynucleotide and determining whether or not the hybridization occurred. A suitable probe and hybridization conditions may be selected according to techniques known in the art.

The primer or probe disclosed herein may be chemically synthesized by a phosphoramidate solid support method or other well-known methods. This nucleic acid sequence may also be modified using many means known in the art. Nonlimiting examples of this modification include methylation, capping, substitution with one or more homologues of natural nucleotide, and modification between nucleotides, for example, modification into an uncharged linker (e.g., methyl phosphonate, phosphotriester, phosphoroamidate, carbamate, etc.) or charged linker (e.g., phosphorothioate, phosphorodithioate, etc.).

"Measurement of protein expression level" may refer to a process that determines the presence and expression level of a protein expressed from the cancer marker gene in a biological sample in order to diagnose cancer. Preferably, the amount of the protein encoded by the gene can be determined using an antibody that binds specifically to the protein. Assays for this measurement include, but are not limited to, Western blotting, ELISA (enzyme linked immunosorbent assay), RIA (radioimmunoassay), radioimmunodiffusion, Ouchterlony immunodiffusion assay, rocket immunoelectrophoresis assay, immunohistostaining assay, immunoprecipitation assay, complement fixation assay, fluorescence activated cell sorter (FACS), protein chip assay, etc.

"An agent for measuring the protein expression level" refers to a molecule that may be used to detect a marker by determining the expression level of a protein from the WLS gene which is a marker whose expression increases when the Wnt signaling pathway is overactivated. Preferably, the agent may be an agent comprising an antibody specific for the protein, but is not limited thereto. Based on the amino acid sequence of the protein expressed from the WLS gene, those skilled in the art can design an antibody specific for the protein.

A monoclonal antibody specific for the protein, which is used in the present invention, may be constructed by a conventional monoclonal antibody construction method known in the art or is commercially available. Furthermore, a polyclonal antibody that recognizes the protein may used instead of the monoclonal antibody, and it may also be constructed by a conventional antiserum construction method known in the art.

The term "kit" may mean a set of compositions and accessories required for a specific purpose. With the respect of the purpose of the present invention, the kit of the present disclosure can diagnose gastric cancer by determining the mRNA expression level of WLS, which is a marker for diagnosing abnormalities in the Wnt signaling pathway, or the expression level of the protein encoded by WLS. The kit of the present disclosure may include not only a primer a probe, or an antibody that selectively recognizes the marker for measuring the expression level of a marker for diagnosing gastric cancer, but also one or more other compositions, solutions or devices suitable for analysis.

"Administration" means introducing the composition of the present invention into a patient by any suitable method. The composition of the present invention may be administered by any general route, as long as it can reach a target tissue. Specifically, the composition used in the present invention may be administered orally, intraperitoneally, intravenously, intramuscularly, subcutaneously, intradermally, intranasally, intrapulmonarily, intrarectally, intracavitally or intrathecally, but is not limited thereto. A treatment in a medical use according to the present invention may comprise administering a pharmaceutically effective amount of the pharmaceutical composition. In the present invention, the effective amount can be determined depending on various factors, including the kind of disease, the severity of the disease, the kinds and contents of active ingredient and other ingredients in the composition, the kind of formulation, the patient's age, body weight, general health condition, sex and diet, the time of administration, the route of administration, the secretion rate of the composition, the period of treatment, and other drugs that are concurrently used. For an adult, the inhibitor of expression of the gene or the inhibitor of activity of the protein may be administered once or several times a day, and in this case, siRNA may be administered at a dose of 0.01 ng/kg to 10 mg/kg, the antisense oligonucleotide for the mRNA of the gene may be administered at a dose of 0.01 ng/kg to 10 mg/kg, the compound may be administered at a dose of 0.1 ng/kg to 10 mg/kg, and the monoclonal antibody for the protein may be administered at a dose of 0.1 ng/kg to 10 mg/kg.

The composition contains, based on the total weight of the composition, 0.1-50 wt% of an antibody against a protein of any one or more of SEQ ID NOs: 1 to 3 as an active ingredient. The composition used in the present invention may further contain a suitable carrier, excipient or diluent according to a conventional method. Carriers, excipients and diluents which may be contained in the composition used in the present invention include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxylbenzoate, talc, magnesium stearate, and mineral oil. The composition used according to the present invention may be formulated as oral dosage forms, including powders, granules, tablets, capsules, suspensions, emulsions, syrup and aerosol, preparations for external application, suppositories, or sterile injectable solutions. Specifically, the composition used in the present invention may be formulated with commonly used diluents or excipients, such as fillers, extenders, binders, wetting agents, disintegrants, surfactants, etc. Solid formulations for oral administration include tablets, pills, powders, granules, capsules and the like, and such solid formulations comprise, in addition to the composition, at least one excipient, for example, starch, calcium carbonate, sucrose, lactose, gelatin or the like. In addition to simple excipients, lubricants such as magnesium stearate or talc may also be used. Liquid formulations for oral administration include suspensions, solutions, emulsions, and syrup, and may contain various excipients, for example, wetting agents, flavoring agents, aromatics and preservatives, in addition to water and liquid paraffin, which are simple diluents that frequently used. Formulations for parenteral administration include sterilized aqueous solutions, non-aqueous solutions, suspensions, emulsions, freeze-dried preparations, and suppositories. As non-aqueous solvents or suspending agents, propylene glycol, polyethylene glycol, plant oils such as olive oil, injectable esters such as ethyl oleate, and the like can be used. As the base of the suppositories, witepsol, Macrogol, Tween 61, cacao butter, laurin fat, glycerogelatin or the like can be used, but is not limited thereto.

In one embodiment of the present invention, the term "screening" means that a substance having any specific property is selected from a candidate group consisting of a plurality of substances by a specific manipulation or evaluation method. With respect of the object of the present invention, the screening of the present invention is a screening method in which a candidate substance is determined as a cancer therapeutic agent when the activity of a protein encoded by the peptide or nucleic acid of the present disclosure is inhibited after the candidate substance for treating cancer is administered to a subject suspected of having cancer or metastasized cancer. Methods such as molecular biological assay, digital imaging, cytological and histological tests may be used to measure the activity of the gene or protein of the present disclosure in a cancer lesion or a tissue suspected of having metastasized cancer cells.

According to one example of the present disclosure, there is provided a pharmaceutical composition for preventing or treating a Wnt signaling pathway-associated disease, the composition containing an antibody specific for a protein represented by an amino acid sequence selected from the group consisting of: (i) an amino acid sequence represented by any one of SEQ ID NOs: 1 to 3; (ii) an amino acid sequence comprising at least 6 consecutive amino acids of the amino acid sequence of any one of SEQ ID NOs: 1 to 3; and (iii) an amino acid sequence having a homology of at least 90% to the amino acid sequence of (i) or (ii).

The present invention provides a pharmaceutical composition for preventing or treating a Wnt signaling pathway-associated cancer, the composition containing an antibody specific for a motif protein of the WLS protein, which is represented by SEQ ID NO: 2.

Preferably, the present disclosure provides a pharmaceutical composition for preventing or treating a Wnt signaling pathway-associated disease, the composition containing an antibody specific for a motif protein of the WLS protein, which is represented by at least one of SEQ ID NOs: 4 to 6, more preferably a motif protein of the WLS protein, which is represented by SEQ ID NO: 6.

The antibody that is provided in the present invention may bind specifically to a specific motif of the WLS protein to prevent overactivation of the Wnt signaling pathway to thereby prevent or treat a disease caused by overactivation of the Wnt signaling pathway.

In the present disclosure, the disease caused by overactivation of the Wnt signaling pathway may be selected, for example, from the group consisting of cancer, retinopathy, macular degeneration, fibrosis, fungal or viral infection, bone or cartilage disease, osteoarthritis, rheumatoid arthritis, neurodegenerative disease, diabetes, a disease of the digestive system, cardiovascular disease and renal disease, but is not limited thereto (Science. 2005 Aug 19;309(5738):1256-9.; Gastroenterology. 2005 Aug;129(2):626-38.; GENES & DEVELOPMENT 19:1596-1611; Trends Cell Biol. 2006 Mar;16(3):151-8. Epub 2006 Feb 7.; J Physiol. 2013 Mar 15;591(Pt 6):1409-32.; IUBMB Life. 2007 Apr-May;59(4-5):316-21.; Organogenesis. 2008 Apr-Jun; 4(2): 55-59.; Mol Pharmacol 82:168-177, 2012; J Cell Biochem. 2012 January ; 113(1): 49-60.; Science. 2010 March 26; 327(5973): 1650-1653.; Life Sciences 89 (2011) 545-554.).

Herein, the cancer may be selected from the group consisting of small-cell lung cancer, non-small-cell lung cancer, breast cancer, prostate cancer, carcinoid, bladder cancer, gastric cancer, pancreatic cancer, liver cancer, colon cancer, rectal cancer, colorectal cancer, renal cancer, head cancer, bone cancer, neck squamous cell carcinoma, esophageal cancer, ovarian cancer, cervical cancer, endometrial cancer, mesothelioma, melanoma, sarcoma, osteosarcoma, liposarcoma, thyroid cancer, desmoids, acute myelocytic leukemia (AML), and chronic myelocytic leukemia (CML). Preferably, the cancer may be gastric cancer, breast cancer, skin cancer, renal cancer or colorectal cancer. More preferably, the cancer may be gastric cancer or colorectal cancer.

The fibrosis may be selected from the group consisting of skin fibrosis; scleroderma; progressive systemic fibrosis; lung fibrosis; muscle fibrosis; kidney fibrosis; glomerulosclerosis; glomerulonephritis; hypertropic scar formation; uterine fibrosis; renal fibrosis; cirrhosis of the liver, liver fibrosis; adhesions, such as those occurring in the abdomen, pelvis, spine or tendons; chronic obstructive pulmonary disease; fibrosis following myocardial infarction; lung fibrosis; scar formation associated with fibrosis and diffuse interstitial lung disease; central nervous system fibrosis, such as fibrosis following stroke; fibrosis associated with neurodegenerative disorders such as Alzheimer's disease or multiple sclerosis; fibrosis associated with proliferative vitreoretinopathy (PVR); restenosis; endometriosis; ischemic disease and radiation fibrosis. Preferably, the fibrosis may be lung fibrosis.

The fungal or viral infection may be Herpes virus, Varicella virus, Epstein-Barr virus, Sindbis virus or Adenovirus infection.

The neurodegenerative disease may be Alzheimer's disease, frontotemporal lobar degeneration, dementia with Lewy bodies, prion disease, Parkinson's disease, Huntington's disease, progressive supranuclear ophthalmoplegia, corticobasal degeneration, multiple system atrophy, amyotrophic lateral sclerosis (ALS), inclusion body myositis, autism, degenerative radiculopathy, diabetic neuropathy, other metabolic neuropathies, endocrine neuropathy, orthostatic hypotension, multiple sclerosis and Charcot-Marie-Tooth disease.

The cardiovascular disease may be cardiac hypertrophy or myocardial infarction.

The renal disease may be selected from the group consisting of cystic kidney disease, acute renal failure, diabetic nephropathy, and ischemic injury.

According to another embodiment of the present disclosure, there is provided a pharmaceutical composition for preventing or treating a Wnt signaling pathway-associated disease, the composition containing any one or more selected from the group consisting of antisense oligonucleotide, siRNA, shRNA and microRNA, which are specific for a gene encoding an amino acid sequence selected from the group consisting of: (i) an amino acid sequence represented by any one of SEQ ID NOs: 1 to 3; (ii) an amino acid sequence comprising at least 6 consecutive amino acids of the amino acid sequence of any one of SEQ ID NOs: 1 to 3; and (iii) an amino acid sequence having a homology of at least 90% to the amino acid sequence of (i) or (ii).

Preferably, the present disclosure provides a pharmaceutical composition for preventing or treating a Wnt signaling pathway-associated disease, the composition containing any one or more selected from the group consisting of antisense oligonucleotide, siRNA, shRNA and microRNA, which are specific for a gene encoding a motif protein of the WLS protein, which is represented by SEQ ID NO: 2.

Preferably, the present disclosure provides a pharmaceutical composition for preventing or treating a Wnt signaling pathway-associated disease, the composition containing any one or more selected from the group consisting of antisense oligonucleotide, siRNA, shRNA and microRNA, which are specific for a gene encoding a motif protein of the WLS protein, which is represented by at least one of SEQ ID NOs: 4 to 6, more preferably a motif protein of the WLS protein, which is represented by SEQ ID NO: 6.

The siRNA, shRNA and microRNA that are provided in the present disclosure may bind specifically to a specific motif of the WLS protein to prevent overactivation of the Wnt signaling pathway to thereby prevent or treat a disease caused by overactivation of the Wnt signaling pathway.

In the present disclosure, the kind of disease caused by overactivation of the Wnt signaling pathway is as described above, and thus the description thereof will be omitted.

According to the present disclosure, there is also provided a pharmaceutical composition for inhibiting angiogenesis, the composition containing an antibody specific for a protein represented by an amino acid sequence selected from the group consisting of: (i) an amino acid sequence represented by any one of SEQ ID NOs: 1 to 3; (ii) an amino acid sequence comprising at least 6 consecutive amino acids of the amino acid sequence of any one of SEQ ID NOs: 1 to 3; and (iii) an amino acid sequence having a homology of at least 90% to the amino acid sequence of (i) or (ii).

Preferably, the present disclosure provides a pharmaceutical composition for inhibiting angiogenesis, the composition containing an antibody specific for a motif protein of the WLS protein, which is represented by SEQ ID NO: 2.

Preferably, the present disclosure provides a pharmaceutical composition for inhibiting angiogenesis, the composition containing an antibody specific for a motif protein of the WLS protein, which is represented by at least one of SEQ ID NOs: 4 to 6, more preferably a motif protein of the WLS protein, which is represented by SEQ ID NO: 6.

The composition that is used in the present invention may prevent overactivation of the Wnt signaling pathway to inhibit angiogenesis caused by overactivation of the Wnt signaling pathway to thereby prevent and/or treat cancer growth or metastasis.

According to the present disclosure, there is also provided a pharmaceutical composition for inhibiting angiogenesis, the composition containing any one or more selected from the group consisting of antisense oligonucleotide, siRNA, shRNA and microRNA, which are specific for a gene encoding an amino acid sequence selected from the group consisting of: (i) an amino acid sequence represented by any one of SEQ ID NOs: 1 to 3; (ii) an amino acid sequence comprising at least 6 consecutive amino acids of the amino acid sequence of any one of SEQ ID NOs: 1 to 3; and (iii) an amino acid sequence having a homology of at least 90% to the amino acid sequence of (i) or (ii).

Preferably, the present disclosure provides a pharmaceutical composition for preventing or treating a Wnt signaling pathway-associated disease, the composition containing any one or more selected from the group consisting of antisense oligonucleotide, siRNA, shRNA and microRNA, which are specific for a gene encoding a motif protein of the WLS protein, which is represented by SEQ ID NO: 2.

Preferably, the present disclosure provides a pharmaceutical composition for preventing or treating a Wnt signaling pathway-associated disease, the composition containing any one or more selected from the group consisting of antisense oligonucleotide, siRNA, shRNA and microRNA, which are specific for a gene encoding a motif protein of the WLS protein, which is represented by at least one of SEQ ID NOs: 4 to 6, more preferably a motif protein of the WLS protein, which is represented by SEQ ID NO: 6.

The composition that is provided in the present invention may prevent overactivation of the Wnt signaling pathway to inhibit angiogenesis caused by overactivation of the Wnt signaling pathway to thereby prevent and/or treat cancer growth or metastasis.

According to still another embodiment of the present disclosure, there is provided a pharmaceutical composition for inhibiting cancer metastasis, the composition containing an antibody specific for a protein represented by an amino acid sequence selected from the group consisting of: (i) an amino acid sequence represented by any one of SEQ ID NOs: 1 to 3; (ii) an amino acid sequence comprising at least 6 consecutive amino acids of the amino acid sequence of any one of SEQ ID NOs: 1 to 3; and (iii) an amino acid sequence having a homology of at least 90% to the amino acid sequence of (i) or (ii).

Preferably, the present invention provides a pharmaceutical composition for inhibiting cancer metastasis, the composition containing an antibody specific for a motif protein of the WLS protein, which is represented by SEQ ID NO: 2.

Preferably, the present invention provides a pharmaceutical composition for inhibiting cancer metastasis, the composition containing an antibody specific for a motif protein of the WLS protein, which is represented by at least one of SEQ ID NOs: 4 to 6, more preferably a motif protein of the WLS protein, which is represented by SEQ ID NO: 6.

The composition that is used in the present invention may prevent overactivation of the Wnt signaling pathway to thereby effectively prevent and/or treat cancer metastasis caused by overactivation of the Wnt signaling pathway.

According to still another embodiment of the present disclosure, there is provided a pharmaceutical composition for inhibiting cancer metastasis, the composition containing any one or more selected from the group consisting of antisense oligonucleotide, siRNA, shRNA and microRNA, which are specific for a gene encoding an amino acid sequence selected from the group consisting of: (i) an amino acid sequence represented by any one of SEQ ID NOs: 1 to 3; (ii) an amino acid sequence comprising at least 6 consecutive amino acids of the amino acid sequence of any one of SEQ ID NOs: 1 to 3; and (iii) an amino acid sequence having a homology of at least 90% to the amino acid sequence of (i) or (ii).

Preferably, the present disclosure provides a pharmaceutical composition for inhibiting cancer metastasis, the composition containing any one or more selected from the group consisting of antisense oligonucleotide, siRNA, shRNA and microRNA, which are specific for a gene encoding a motif protein of the WLS protein, which is represented by SEQ ID NO: 2.

Preferably, the present disclosure provides a pharmaceutical composition for inhibiting cancer metastasis, the composition containing any one or more selected from the group consisting of antisense oligonucleotide, siRNA, shRNA and microRNA, which are specific for a gene encoding a motif protein of the WLS protein, which is represented by at least one of SEQ ID NOs: 4 to 6, more preferably a motif protein of the WLS protein, which is represented by SEQ ID NO: 6.

The composition that is provided in the present invention may prevent overactivation of the Wnt signaling pathway to thereby prevent and/or treat cancer metastasis caused by overactivation of the Wnt signaling pathway.

According to still another embodiment of the present disclosure, there is provided a pharmaceutical composition for diagnosing cancer or cancer metastasis, the composition containing an antibody specific for a protein represented by an amino acid sequence selected from the group consisting of: (i) an amino acid sequence represented by any one of SEQ ID NOs: 1 to 3; (ii) an amino acid sequence comprising at least 6 consecutive amino acids of the amino acid sequence of any one of SEQ ID NOs: 1 to 3; and (iii) an amino acid sequence having a homology of at least 90% to the amino acid sequence of (i) or (ii).

The present invention provides a pharmaceutical composition for diagnosing cancer or cancer metastasis, the composition containing an antibody specific for a motif protein of the WLS protein, which is represented by SEQ ID NO: 2.

Preferably, the present disclosure provides a pharmaceutical composition for diagnosing cancer or cancer metastasis, the composition containing an antibody specific for a motif protein of the WLS protein, which is represented by at least one of SEQ ID NOs: 4 to 6, more preferably a motif protein of the WLS protein, which is represented by SEQ ID NO: 6.

Cancer cells that are developed or metastasized due to overactivation of the Wnt signaling pathway show an increased expression level of the WLS protein. Thus, the composition for diagnosis according to the present disclosure may be used to detect a pathological marker of cancer growth or metastasis, and enables cancer growth or metastasis to be diagnosed by quantitatively analyzing the expression level of WLS protein. More preferably, the composition of the present disclosure makes it possible to diagnose the growth or metastasis of gastric cancer or colorectal cancer.

The disclosure also provides a pharmaceutical composition for diagnosing cancer or cancer metastasis, the composition containing a primer or probe specific for a gene encoding an amino acid sequence selected from the group consisting of: (i) an amino acid sequence represented by any one of SEQ ID NOs: 1 to 3; (ii) an amino acid sequence comprising at least 6 consecutive amino acids of the amino acid sequence of any one of SEQ ID NOs: 1 to 3; and (iii) an amino acid sequence having a homology of at least 90% to the amino acid sequence of (i) or (ii).

The present disclosure also provides a pharmaceutical composition for diagnosing cancer or cancer metastasis, the composition containing a primer or probe specific for a gene encoding a motif protein of the WLS protein, which is represented by SEQ ID NO: 2.

The present disclosure also provides a pharmaceutical composition for diagnosing cancer or cancer metastasis, the composition containing a primer or probe specific for a gene encoding a motif protein of the WLS protein, which is represented by at least one of SEQ ID NOs: 4 to 6, more preferably a motif protein of the WLS protein, which is represented by SEQ ID NO: 6.

As described above, cancer cells that are developed or metastasized due to overactivation of the Wnt signaling pathway show an increased expression level of the WLS protein. Thus, the composition enables cancer growth or metastasis to be diagnosed by quantitatively analyzing the expression level of mRNA encoding the WLS protein. The present disclosure makes it possible to diagnose the growth or metastasis of gastric cancer or colorectal cancer.

According to still another embodiment of the present disclosure, there is provided a method for providing information about cancer or cancer metastasis, the method comprising measuring the expression level of a protein in a subject by use of an antibody specific for the protein represented by an amino acid sequence selected from the group consisting of: (i) an amino acid sequence represented by any one of SEQ ID NOs: 1 to 3; (ii) an amino acid sequence comprising at least 6 consecutive amino acids of the amino acid sequence of any one of SEQ ID NOs: 1 to 3; and (iii) an amino acid sequence having a homology of at least 90% to the amino acid sequence of (i) or (ii).

According to the present disclosure, there is also provided a method for providing information about cancer or cancer metastasis, the method comprising measuring the mRNA expression level of WLS gene in a subject by use of a primer or probe specific for a gene encoding an amino acid sequence selected from the group consisting of: (i) an amino acid sequence represented by any one of SEQ ID NOs: 1 to 3; (ii) an amino acid sequence comprising at least 6 consecutive amino acids of the amino acid sequence of any one of SEQ ID NOs: 1 to 3; and (iii) an amino acid sequence having a homology of at least 90% to the amino acid sequence of (i) or (ii).

According to the present disclosure, there is furthermore provided a method for screening a cancer therapeutic agent, the method comprising determining that a candidate substance for cancer treatment is a co-agent for cancer therapy when expression of WLS protein is inhibited in a case where an antibody specific for a WLS (Wntless) motif protein represented by any one of SEQ ID NOs: 1 to 3 is administered in combination with the candidate substance, compared to a case where the WLS antibody is administered alone.

According to the present disclosure, there is also provided a method for screening a cancer therapeutic agent, the method comprising determining that a candidate substance for cancer treatment is a co-agent for cancer therapy when expression of WLS protein is inhibited in a case where any one or more selected from the group consisting of antisense oligonucleotide, siRNA, shRNA and microRNA, which are specific for a gene encoding a WLS (Wntless) motif protein represented by any one of SEQ ID NOs: 1 to 3, are administered in combination with the candidate substance, compared to a case where the WLS antibody is administered alone.

According to the present disclosure, there is also provided a protein represented by an amino acid sequence selected from the group consisting of: (i) an amino acid sequence represented by any one of SEQ ID NOs: 1 to 3; (ii) an amino acid sequence comprising at least 6 consecutive amino acids of the amino acid sequence of any one of SEQ ID NOs: 1 to 3; and (iii) an amino acid sequence having a homology of at least 90% to the amino acid sequence of (i) or (ii).

According to the present disclosure, there is also provided an antibody specific for a protein represented by an amino acid sequence selected from the group consisting of: (i) an amino acid sequence represented by any one of SEQ ID NOs: 1 to 3; (ii) an amino acid sequence comprising at least 6 consecutive amino acids of the amino acid sequence of any one of SEQ ID NOs: 1 to 3; and (iii) an amino acid sequence having a homology of at least 90% to the amino acid sequence of (i) or (ii).

Disclosed is also a gene encoding a protein represented by an amino acid sequence selected from the group consisting of: (i) an amino acid sequence represented by any one of SEQ ID NOs: 1 to 3; (ii) an amino acid sequence comprising at least 6 consecutive amino acids of the amino acid sequence of any one of SEQ ID NOs: 1 to 3; and (iii) an amino acid sequence having a homology of at least 90% to the amino acid sequence of (i) or (ii).

Hereinafter, each step of the present disclosure will be described in detail.

### [Advantageous Effects]

The antibody that is used in the present invention can recognize a specific motif of the WLS protein, which regulates the secretion of the Wnt protein in Wnt signaling, so as to inhibit overactivation of Wnt signaling to thereby effectively prevent or treat a disease associated with the Wnt signaling pathway.

### [Description of Drawings]

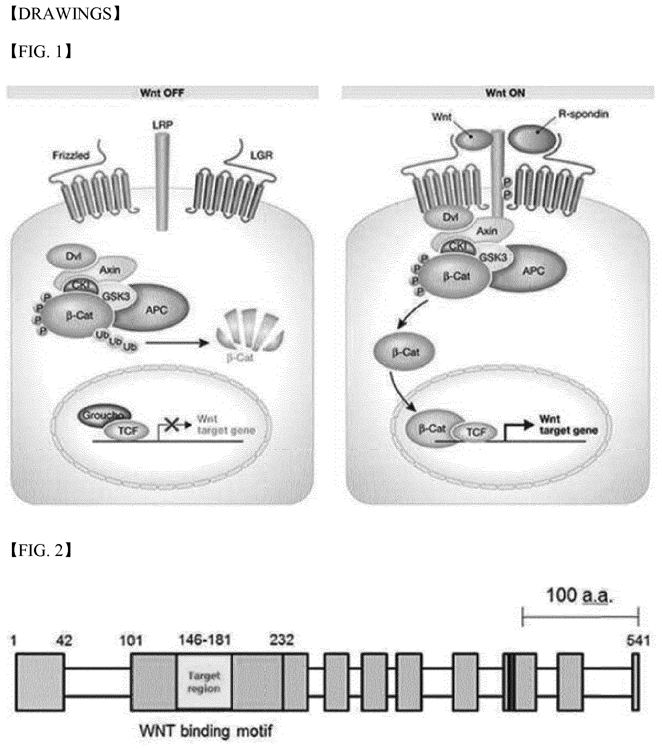

FIG. 1 is a schematic view showing the WLS protein before or after Wnt binding according to an example of the present disclosure.
FIG. 2 is a schematic view showing the Wnt binding motif of the WLS protein according to an example of the present disclosure.
FIG. 3 shows the results of measuring the correlation between WLS expression and gastric cancer prognosis according to an example of the present disclosure.
FIG. 4 shows the WLS protein (SEQ ID NO.: 7) divided into five peptides according to an example of the present disclosure.
FIG. 5a shows the results of measuring the responsiveness of WLS#2, WLS#3, WLS#4 and WLS#5 antibodies to the WLS protein in an AGS gastric cancer cell line according to an example of the present disclosure.
FIG. 5b shows the results of examining the reduction in WLS detection ability of an antibody used together with a peptide, according to an example of the present disclosure.
FIG. 6a shows the results of examining the effect of siWLS #1 on the inhibition of FLAG-WLS expression according to an example of the present disclosure.
FIG. 6b shows the results of examining the binding of a monoclonal antibody to immunoprecipitated FLAG-WLS protein according to an example of the present disclosure.
FIG. 7a shows the results of examining the position of WLS in an AGS gastric cancer cell line by immunostaining according to an example of the present disclosure.
FIG. 7b shows the results of analyzing the expression patterns of WLS in intracellular fractions by Western-blotting according to an example of the present disclosure.
FIG. 7c shows the results of examining the position of WLS in an AGS gastric cancer cell line by immunostaining according to an example of the present disclosure.
FIG. 7d shows the results of analyzing the binding of WLS#3 antibody to endogenous WLS in an AGS gastric cancer cell line by FACS analysis according to an example of the present disclosure.
FIG. 7e shows the results of analyzing the binding of WLS#4 antibody to endogenous WLS in an AGS gastric cancer cell line by FACS analysis according to an example of the present disclosure.
FIG. 8 shows the results of analyzing the expression levels of WLS, Axin2 and CyclinDl in various gastric cancer cell lines according to an example of the present disclosure.
FIG. 9 shows the results of analyzing cell viability when an AGS gastric cancer cell line was treated with 1 µg/ml of a WLS monoclonal antibody, according to an example of the present disclosure.
FIG. 10a shows the results of analyzing reduced cell viability when an AGS gastric cancer cell line was treated with 4 µg/ml of a WLS monoclonal antibody, according to an example of the present disclosure.
FIG. 10b shows the results of observing abnormal cell morphology when an AGS gastric cancer cell line was treated with a WLS monoclonal antibody, according to an example of the present disclosure.
FIG. 11 shows the results of examining the effects of WLS#3 and WLS#4 antibodies on the inhibition of cancer cell survival according to an example of the presentdisclosure disclosure.
FIG. 12 shows the results of examining cell viability when two gastric cancer lines showing different expression patterns of the WLS gene were treated with a monoclonal antibody, according to an example of the present disclosure.
FIG. 13a shows the results of measuring the expression levels of WLS in MKN74 and NI3-3 cells by qRt-PCR according to an example of the present disclosure.
FIG. 13b shows the results of measuring the expression levels of WLS in MKN74 and NI3-3 by Western blotting according to an example of the present disclosure.
FIG. 13c shows the results of measuring the expression levels of Wnt target genes in MKN74 and NI3-3 cells according to an example of the present disclosure.
FIG. 14 shows the results of examining cell viability when MKN74 and NI3-3 cells were treated with a WLS monoclonal antibody, according to an example of the present disclosure.
FIG. 15 shows the results of examining the change in cell mobility when expression of WLS was inhibited, according to an example of the present disclosure.
FIG. 16 shows the results of examining the effect of a WLS#4 monoclonal antibody on the inhibition of cancer cell mobility according to an example of the present disclosure.
FIG. 17 shows the results of examining the effect of a WLS#3 monoclonal antibody on the inhibition of cancer cell mobility according to an example of the present invention.
FIG. 18 shows the results of FACS analysis that separated MKN74 cells based on the expression level of WLS, according to an example of the present disclosure.
FIG. 19 shows the results of FACS analysis that separated AGS cells based on the expression level of CD44, according to an example of the present disclosure.
FIG. 20 shows the results of analyzing the expression level of WLS in AGS cells having high CD44 expression, according to an example of the present disclosure.
FIG. 21 shows the results of analyzing the acetylation degree of β-catenin in colorectal cancer cells after treatment with a WLS#4 monoclonal antibody according to an example of the present disclosure.
FIG. 22a shows the results of measuring the tumor size of MKN74 cells after treatment with a WLS#4 monoclonal antibody according to an example of the present disclosure.
FIG. 22b shows the results of measuring the tumor size of MKN74 cells after treatment with a WLS#3 monoclonal antibody according to an example of the present invention.
FIG. 23 shows the results of examining the mRNA expression levels of WLS in p-MKN74 cells and s-MKN74 with metabolic starvation according to an example of the present disclosure.
FIG. 24 shows the results of measuring the tumor sizes of p-MKN74 cells and s-MKN74 with metabolic starvation according to an example of the present disclosure.
FIG. 25a shows the results of measuring the tumor sizes of p-MKN74 cells and s-MKN74 after treatment with WLS#3 and WLS#4 monoclonal antibodies according to an example of the present disclosure.
FIG. 25b shows the results of measuring the tumor size of p-MKN74 cells after treatment with WLS#3 and WLS#4 monoclonal antibodies according to an example of the present disclosure.
FIG. 25c shows the results of measuring the tumor size of s-MKN74 after treatment with WLS#3 and WLS#4 monoclonal antibodies according to an example of the present disclosure.
FIG. 26 shows the results of examining the expression level of β-catenin in MCF7 cells after treatment with a WLS#4 monoclonal antibody according to an example of the present disclosure.
FIG. 27 shows the results of examining the expression level of β-catenin in WiDr cells after treatment with a WLS#4 monoclonal antibody according to an example of the present disclosure.
FIG. 28 shows the results of examining the expression level of β-catenin in A431 cells after treatment with a WLS#4 monoclonal antibody according to an example of the present disclosure.
FIG. 29 shows the results of examining the expression level of β-catenin in transfected MCF7 cells after treatment with a WLS#4 monoclonal antibody according to an example of the present disclosure.
FIG. 30 shows the results of examining the expression level of β-catenin in transfected WiDr cells after treatment with a WLS#4 monoclonal antibody according to an example of the present disclosure.
FIG. 31 shows the results of examining the expression level of β-catenin in transfected A431 cells after treatment with a WLS#4 monoclonal antibody according to an example of the present disclosure.

### [Best Mode]

The present disclosure provides a pharmaceutical composition for preventing or treating a Wnt signaling pathway-associated disease, the composition containing an antibody specific for a protein represented by an amino acid sequence selected from the group consisting of: (i) an amino acid sequence represented by any one of SEQ ID NOs: 1 to 3; (ii) an amino acid sequence comprising at least 6 consecutive amino acids of the amino acid sequence of any one of SEQ ID NOs: 1 to 3; and (iii) an amino acid sequence having a homology of at least 90% to the amino acid sequence of (i) or (ii).

### [Mode for Invention]

Hereinafter, the present disclosure will be described in further detail. It will be obvious to those skilled in the art that these examples are for illustrative purposes only and are not intended to limit the scope of the present invention.

### Example 1: Identification of WLS Gene

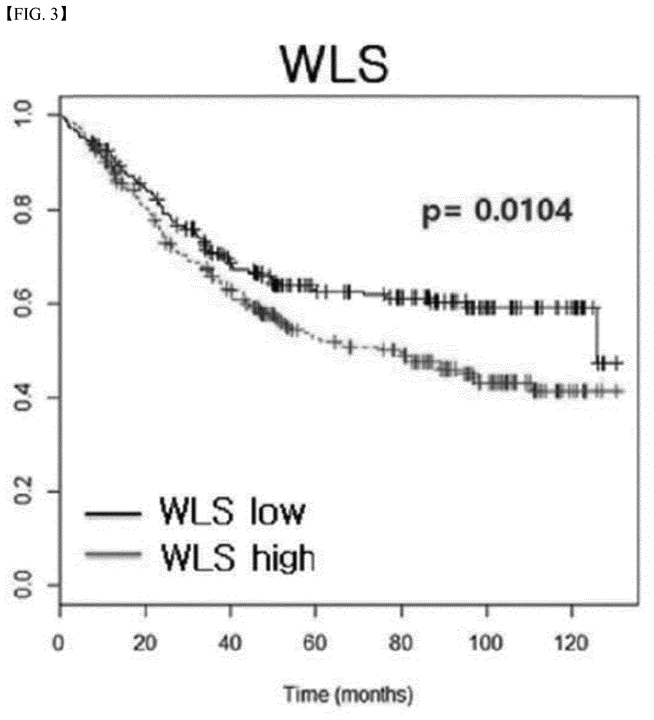

In order to screen target genes in the early stage of gastric cancer, the normal tissue and cancer tissue of 78 early gastric cancer patients who underwent gastrectomy were comparatively analyzed by a microarray. The results of the analysis indicated that a difference in prognosis appeared in the group in which the expression level of a specific gene was high or low. In particular, in the case of the WLS gene, the recurrence of cancer more occurred in the group in which the WLS gene was highly expressed, and the prognosis was bad in the group. In order to confirm the statistical significance of such results, 506 gastric cancer patients independent of the above group were divided into a group in which the expression of WLS was high and a group in which the expression of WLS was low, and the prognosis of the patients was observed. As a result, it was shown that the group in which WLS was highly expressed had a higher hazard ratio, and this difference in hazard ratio was determined to be statistically significant, based on Cox analysis performed according to age, sex and the degree of cancer progression (stage of cancer progression) (FIG. 3).

### Example 2: Construction of WLS Monoclonal Antibodies

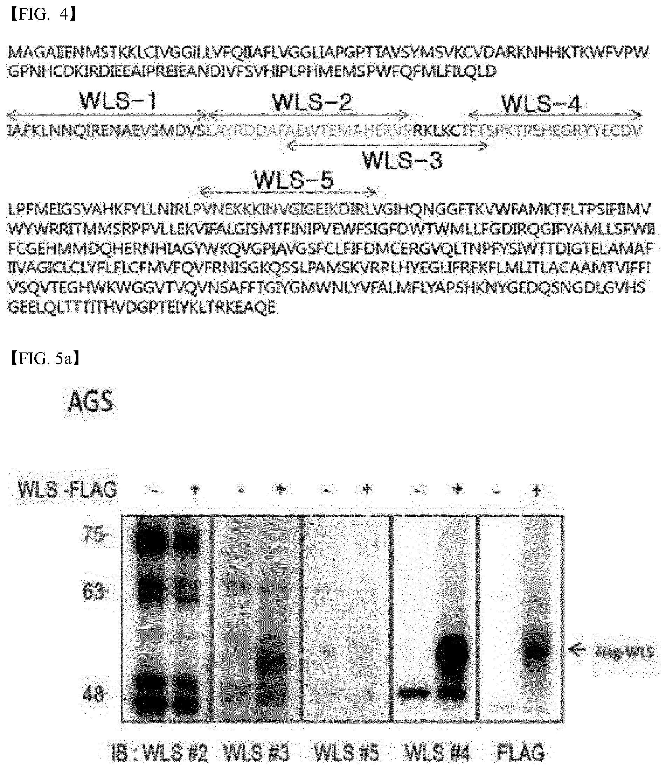

Among portions of the WLS protein structure, which can act as antigens (targeting a portion known as the Wnt-binding region), a total of five peptides (peptide Nos. 1, 2, 3, 4 and 5) were selected. The selected peptides are shown in FIG. 4 and Table 1 below.

**Table 1**

| Peptide Nos. | Amino acid positions | Amino acid sequences |
|---|---|---|
| Peptide No. 1 (SEQ ID NO: 8) | 118-137 | IAFKLNNQIRENAEVSMDVS |
| Peptide No. 2 (SEQ ID NO: 9) | 138-157 | LAYRDDAFAEWTEMAHERVP |
| Peptide No. 3 (SEQ ID NO: 2) | 146-165 | AEWTEMAHERVPRKLKCTFT |
| Peptide No. 4 (SEQ ID NO: 3) | 163-181 | TFTSPKTPEHEGRYYECDV |
| Peptide No. 5 (SEQ ID NO: 10) | 202-222 | PVNEKKKINVGIGEIKDIRL |

Construction of antibodies for the five peptides was attempted. Because peptide No. 1 was not produced, production of an antibody for peptide No. 1 was impossible. Using peptide Nos. 2 to 5, a total of four monoclonal antibodies were constructed. The name of each of the antibodies is shown in Table 2 below.

**Table 2**

| Antibody names (monoclonal antibodies) | Peptide Nos. |
|---|---|
| WLS#2 | Peptide No. 2 |
| WLS#3 | Peptide No. 3 (SEQ ID NO: 2) |
| WLS#4 | Peptide No. 4 (SEQ ID NO: 3) |
| WLS#5 | Peptide No. 5 |

### Example 3: Verification of WLS Monoclonal Antibodies

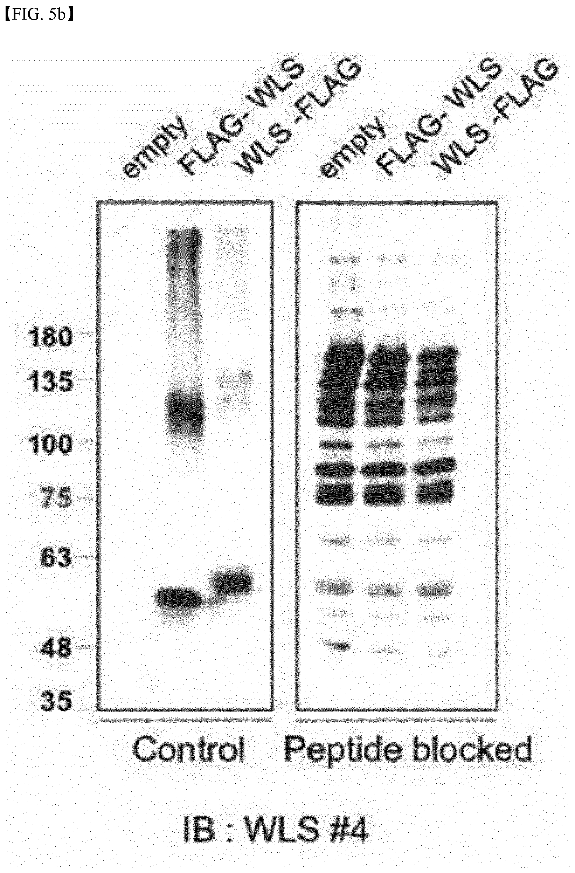

The whole sequence of human WLS (NM_024911) was cloned into a pSG5-KF2M1 (FLAG tag in front) plasmid vector and a pSG-KM1F2 (FLAG tag at back) plasmid vector, and a pSG5-empty vector was used as a negative control. FLAG-WLS was expressed in an AGS gastric cancer cell line, and then analyzed by Western blotting. 15 µg of the cell lysate sample was electrophoresed on SDS-PAGE gel, and WLS#2 to WLS#5 monoclonal antibodies were diluted at a ratio of 1:500 vol% with 5 wt% skim milk before use. It was shown that WLS#2 to WLS#5 antibodies detected the FLAG-tagged WLS protein (FIG. 5a). It was observed that this specific reaction was not detected on Western blotting when the WLS#4 antibody was used for treatment together with the peptide (FIG. 5b).

### Example 4: Analysis of Endogenous WLS Protein

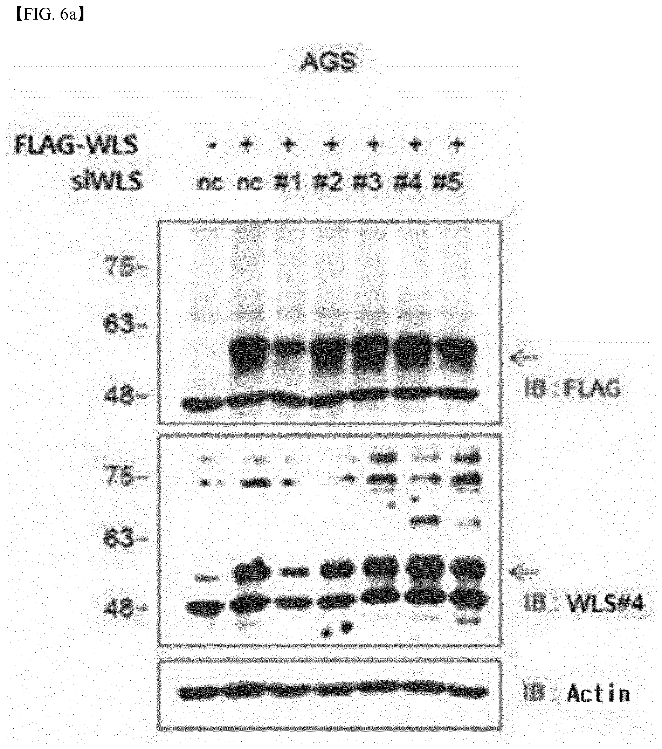

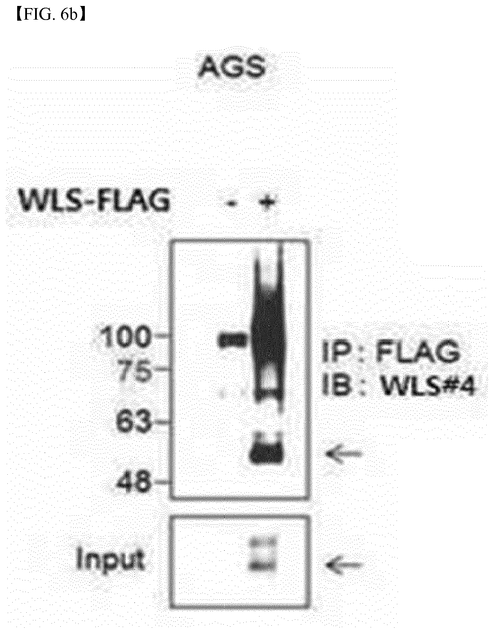

Five siRNAs targeting the human WLS gene were constructed (using Genolution pre-designed siRNA service). The results of Western blotting analysis using the WLS#4 antibody indicated that siWLS #1 (sense 5' GUCAUCUUCUUCAUCGUUAUU 3' (SEQ ID NO: 11); antisense 5' UAACGAUGAAGAAGAUGACUU 3' (SEQ ID NO: 12) of these siRNAs reduced the expression of FLAG-WLS (FIG. 6a). Furthermore, the FLAG-WLS protein was immunoprecipitated using FLAG-M2 beads, and then specific binding was examined using the WLS monoclonal antibody (WLS#4) (FIG. 6b).

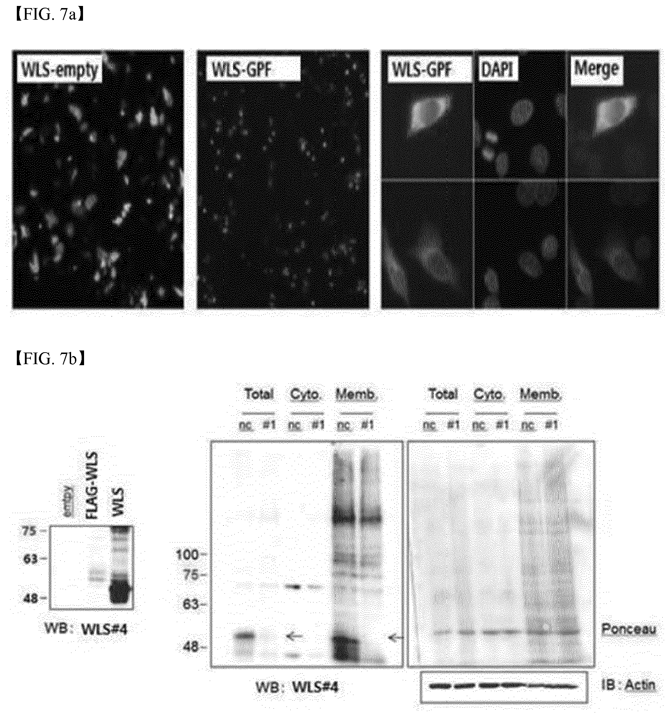

WLS was cloned into a pEGFP-N1 plasmid vector and expressed in an AGS gastric cancer cell line, and the position thereof in the cells was examined. The results of immunofluorescence staining of the cells using the WLS#4 antibody indicated that WLS was widely distributed in the cytosol (FIG. 7a). Furthermore, untagged WLS protein was expressed with FLAG-WLS and compared, and as a result, the position of endogenous WLS on Western blotting could be predicted. Using the siRNA constructed and analyzed as described above, WLS was lowly expressed in an AGS gastric cancer cell line, and then the intracellular fractions were divided into the cytosol and the cell membrane, and the expression pattern of WLS was analyzed by Western blotting. As a result, it was shown that endogenous WLS was expressed in the cell membrane fraction (FIG. 7b).

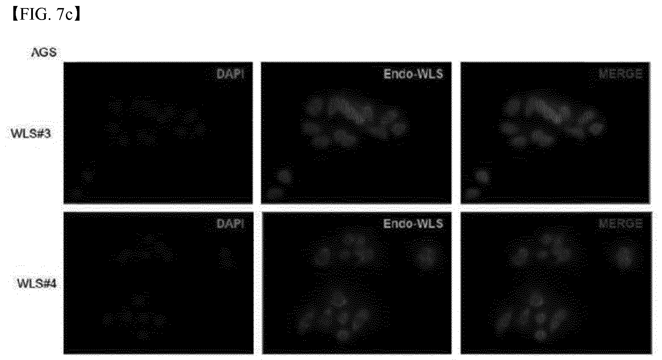

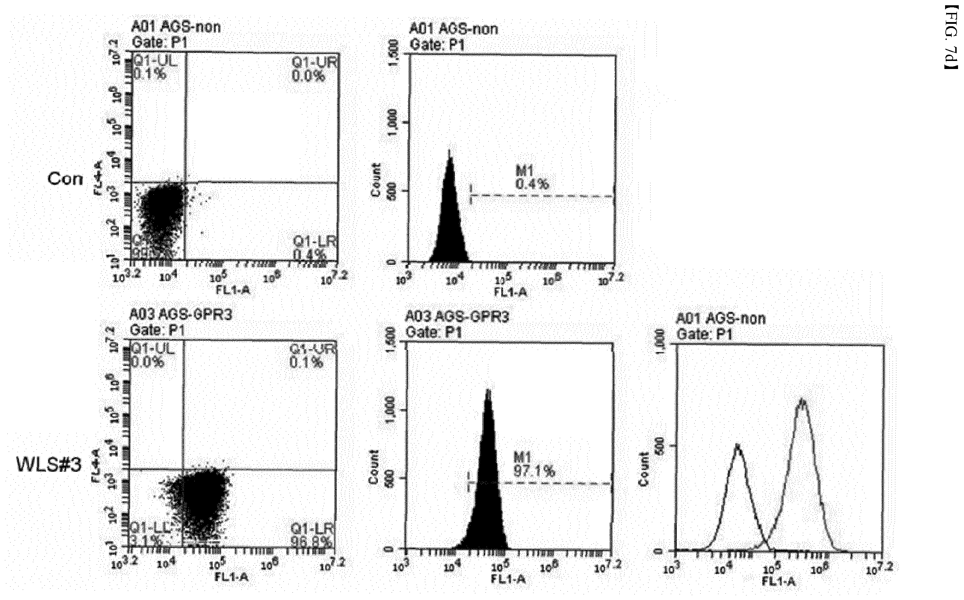

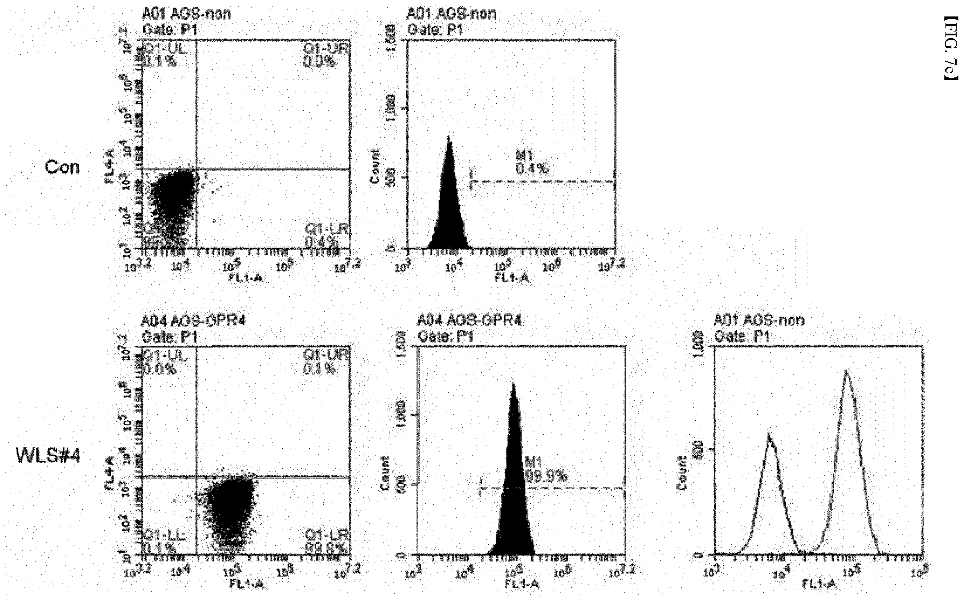

Similarly to the expression patterns of the pEGFP-WLS protein as described above, the AGS gastric cancer cell line was examined by fluorescence staining using the WLS#3 and WLS#4 antibodies, and as a result, it was shown that the endogenous WLS protein was present in the cell membrane (FIG. 7c). Furthermore, the cells were analyzed by FACS analysis, and as a result, it was shown that the WLS#3 and WLS#4 antibodies did bind specifically to the endogenous WLS in the AGS gastric cancer cell line (FIGS. 7d and 7e).

### Example 5: Analysis of Gastric Cancer Cell Line

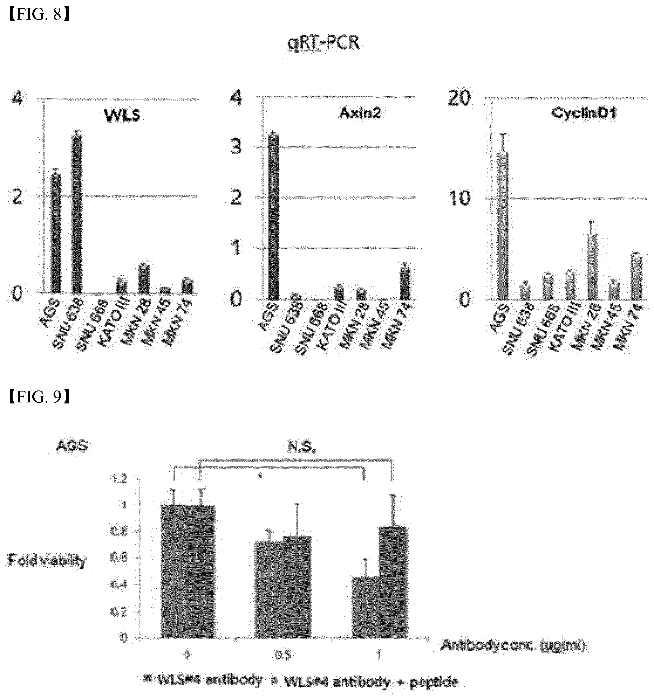

In order to apply the above-constructed monoclonal antibodies to cell experiments, gastric cancer cells were selected, and RNA was extracted from the selected AGS, SNU-638, SNU-668, KATOIII, MKN28, MKN45 and MKN74 cell lines. cDNA was synthesized from the extracted RNA. Then, using a qRT-PCR technique, the mRNA expression levels of Axin2 and CyclinDl (which are WLS- and Wnt-targeting genes) in the cells were analyzed. As a result, it was shown that the expression levels of WLS, Axin2 and CyclinDl in the AGS cell line were commonly high, and that these expression levels were commonly low in the SNU-668, KATOIII, MKN28, MKN45 and MKN74 cells. Exceptionally, in the SNU-638 cells, it was shown that the expression level of WLS was high, but the expression level of the Wnt-targeting gene was low (FIG. 8).

### Example 6: Examination of the Ability of WLS Monoclonal Antibodies to Reduce Cell Growth

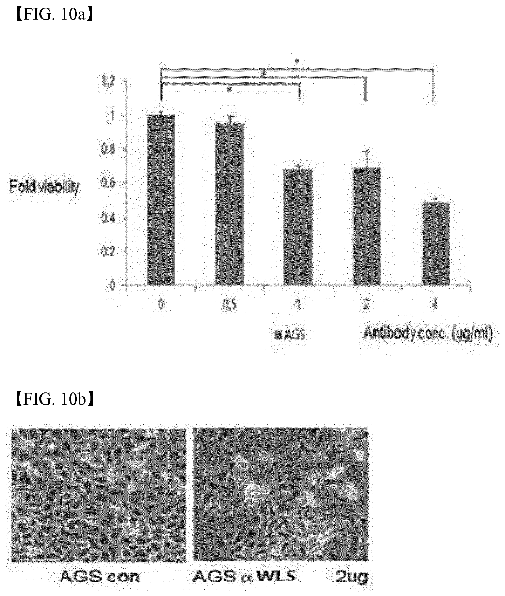

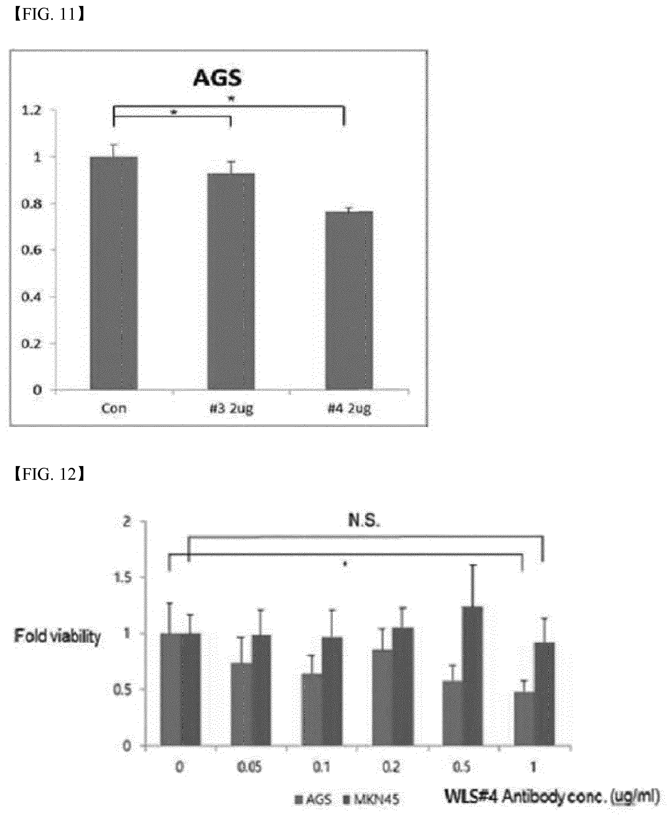

The AGS gastric cancer cell line having high expression levels of WLS and Wnt-targeting genes was treated with 0, 0.5 and 1 µg/ml of the WLS monoclonal antibody (WLS#4), and after 48 hours, the viability of the cells was analyzed by an MTT assay (FIG. 9). As a result, when the cells were treated with 1 µg/ml of the antibody, the viability thereof decreased. Furthermore, when the specificity of 1 µg/ml of the antibody was reduced by the peptide, the viability of the cells did not decrease, indicating that the specific binding of the antibody leads to a decrease in the viability of the cells. Additionally, in order to examine the ability of the monoclonal antibody to reduce cell viability, the cells were treated with the antibody while the concentration of the antibody was increased until the cell viability decreased to 50 vol%. As a result, it was shown that, at a WLS#4 antibody concentration of about 4 µg/ml, a decrease in cell viability of about 50 vol% appeared (FIG. 10a). Furthermore, when the AGS gastric cancer cell line was treated with the WLS monoclonal antibody (WLS#4), abnormal morphology appeared, unlike the control group (FIG. 10b). The cells were treated with 2 µg/ml of each of the WLS monoclonal antibodies #3 and #4, and the viability of the cells was analyzed, and as a result, it was shown that the WLS monoclonal antibodies #3 and #4 all significantly reduced the viability of the cancer cells (FIG. 11).

### Example 7: Examination of Specific Binding of WLS Monoclonal Antibody and the Ability to Reduce Cell Viability

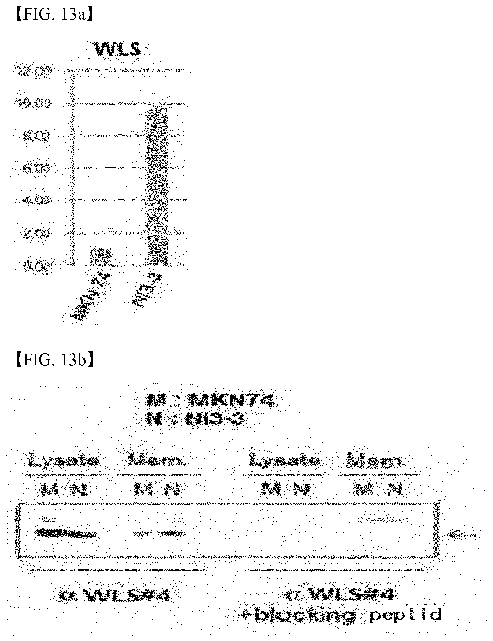

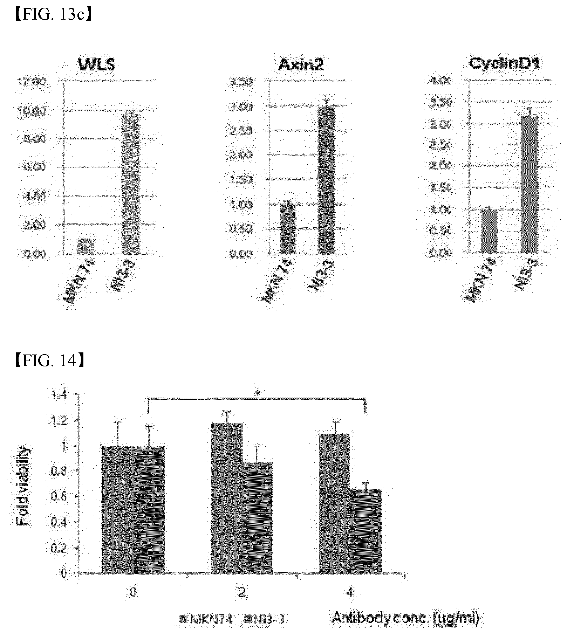

Using AGS gastric cancer cells having a high expression level of the WLS genes and MKN45 cells having a low expression level of the WLS gene, the cells were treated with the WLS#4 monoclonal antibody, and the viability of the cells was analyzed. As a result, the monoclonal antibody was effective even when WLS was highly expressed (FIG. 12). Because this phenomenon is attributable to the features of the AGS and MKN45 cells themselves, a cell model having the same genetic background was prepared. Specifically, MKN74 cells having a low expression level of the WLS gene were injected into the heart of a rat, and the cells were allowed to metastasize to the brain of the rat, after which the MKN74 cells grown in the brain were cultured and named "NI3-3". The expression levels of the WLS gene in the two cell types were analyzed by qRt-PCR and Western blotting using the WLS#4 antibody, and as a result, it was shown that the expression level of WLS in the cells that metastasized to the brain cells was higher (FIGS. 13a and 13b). Furthermore, it was shown that the activities of WLS, Axin2 and CyclinDl genes (which are intracellular Wnt-targeting genes) in the metastasized cells increased compared to those in the original MKN74 cells (FIG. 13c). The ability of the WLS#4 monoclonal antibody to reduce cell viability was examined in the same manner as described above, and as a result, it was shown that the cell viability of only the NI3-3 cell line having a high expression level of WLS was reduced, and it was shown by a peptide inhibitory experiment that this reduction in cell viability was caused by the specific binding of the antibody (FIG. 14).

### Example 8: Examination of the Ability of WLS Monoclonal Antibodies to Reduce Cell Migration

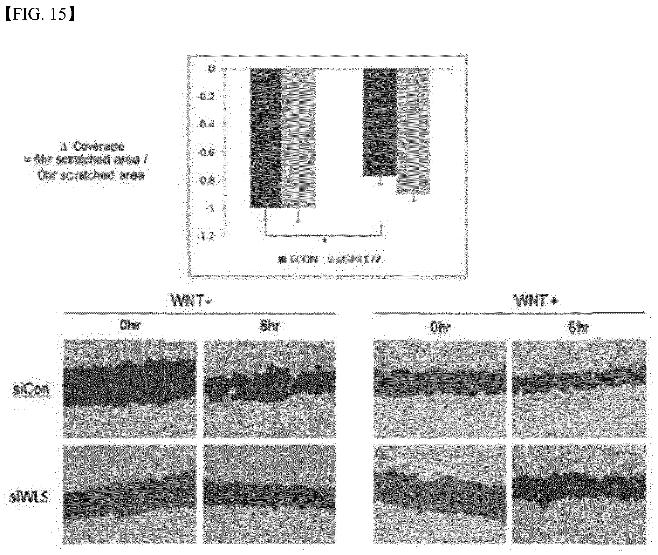

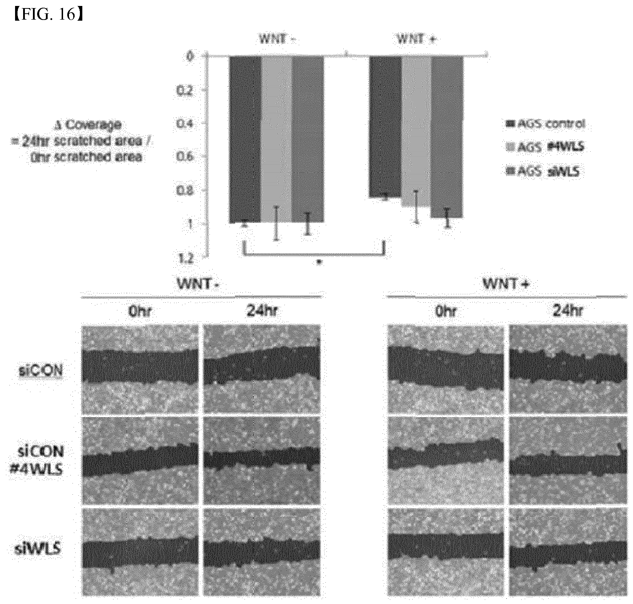

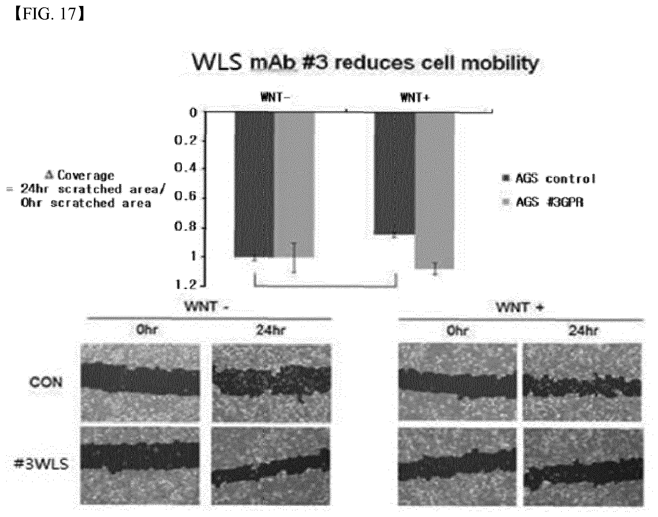

The Wnt gene was expressed in the AGS gastric cancer cell line, and then the mobility of the cells was examined by a cell migration experiment (scratch and healing). The AGS gastric cancer cells were cultured to confluency in a culture dish, and then the cell monolayer was linearly scratched using a 200-µl tip, after which the area in which the cells migrated for 6 hours was calculated. As a result, it was shown that when the expression of WLS was reduced using siWLS #1, the mobility of the cells was reduced (FIG. 15). In addition, using siRNA as a control, the ability of the WLS#4 monoclonal antibody to reduce cell mobility was measured, and as a result, it was shown that the mobility of the cells was also reduced in the group treated with the WLS#4 monoclonal antibody (FIG. 16), similar to the group in which the expression of WLS was reduced by siRNA. In addition, similarly to the WLS#4 monoclonal antibody, it was shown that the WLS#3 monoclonal antibody also reduced the mobility of the cells when the Wnt gene in the cancer cells was expressed (FIG. 17).

### Example 9: FACS Analysis of WLS Monoclonal Antibody

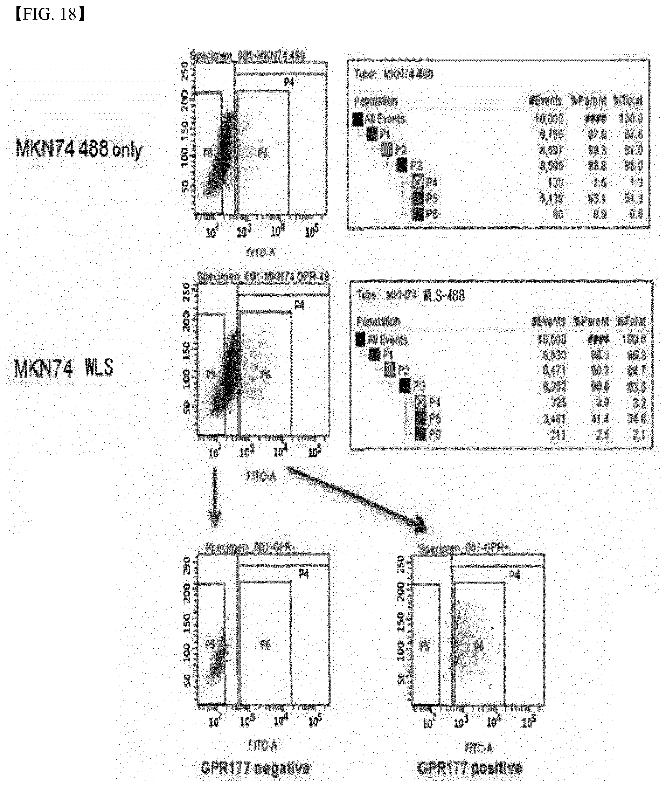

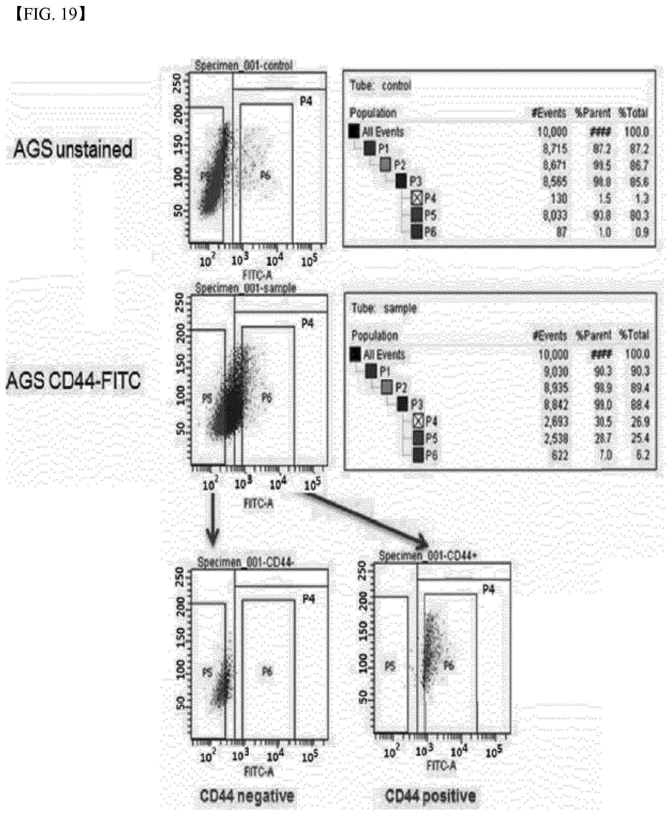

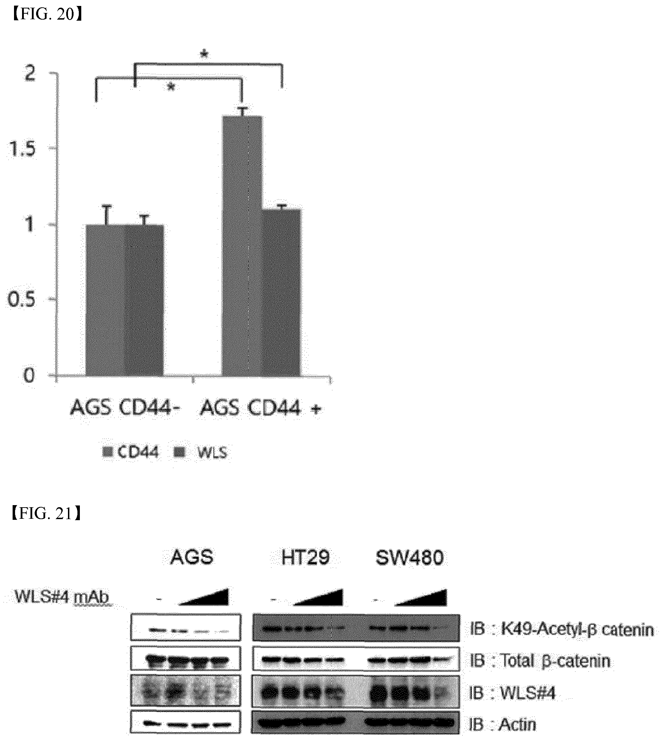

The results of the above Examples indicated that the high expression of WLS can adversely affect the prognosis of the gastric cancer patients and can lead to the increase in metastasis ability in the cell experiment. Thus, in order to analyze the features of cells in which WLS is highly expressed, FACS analysis was performed. Through FACS analysis using the WLS#4 monoclonal antibody, cells having a high expression level of WLS and cells having a low expression level of WLS were separated from MKN74 cells (FIG. 18). Moreover, using the protein CD44 that determines the tumorigenicity of gastric cancer cells, AGS cells were analyzed by FACS analysis (FIG. 19). As a result, it was shown that the expression level of WLS in the AGS cells having a high level of CD44 was also high (FIG. 20).

### Example 10: Inhibition of Signaling of Colorectal Cancer caused by Overactivation of Wnt Signaling Pathway

The gastric cancer cell line AGS and the HT29 and SW480 colorectal cancer lines caused by overactivation of the Wnt signaling pathway were treated with the WLS#4 monoclonal antibody constructed in Example 2, and then a change in the signaling was observed. Specifically, each of the cell lines was treated with 0, 1, 2 and 4 µg/ml of the WLS#4 monoclonal antibody for 48 hours, and then the acetylation of the major transcriptional regulator β-catenin of the Wnt signaling pathway was analyzed by Western blotting. As a result, it was shown that the acetylation of β-catenin that increased due to overactivation of the Wnt signaling pathway was reduced by treatment with the WLS#4 monoclonal antibody (FIG. 21).

### Example 11: Examination (1) of Anticancer Effect of WLS Monoclonal Antibodies

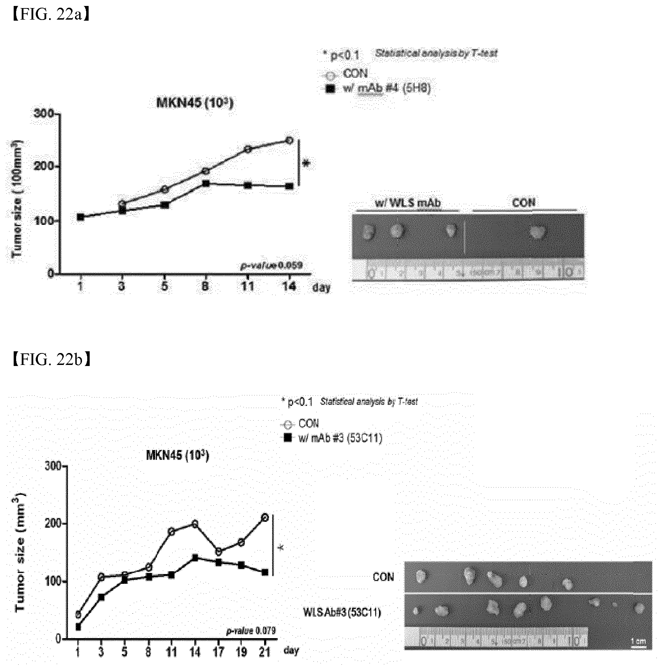

A total of 1 x 10⁴ MKN45 cells mixed with Matrigel at a ratio of 1:2 (v/v) were injected into the subcutaneous tissue of immunodeficient nude mice to form a gastric tumor. When the volume of the tumor reached 100 mm³ after about 2 weeks, the WLS monoclonal antibodies constructed in Example 2 were injected through the tail veins. The WLS#4 monoclonal antibody was injected at a concentration of 5 mg/kg six times for 2 weeks (n=8), and the WLS#3 monoclonal antibody was injected at a concentration of 5 mg/kg nine times for 3 weeks (n=20). During the period from the day on which the monoclonal antibody was first injected to two days following the day on which the monoclonal antibody was finally injected, the volume of the tumor was measured using calipers according to the following equation: short axis² x long axis/2. As a result, it was shown that the tumor size in the test group treated with the WLS#3 or WLS#4 monoclonal antibody significantly decreased compared to the control group not treated with the antibody (FIGS. 22a and 22b).

### Example 12: Examination (2) of Anticancer Effect of WLS Monoclonal Antibodies

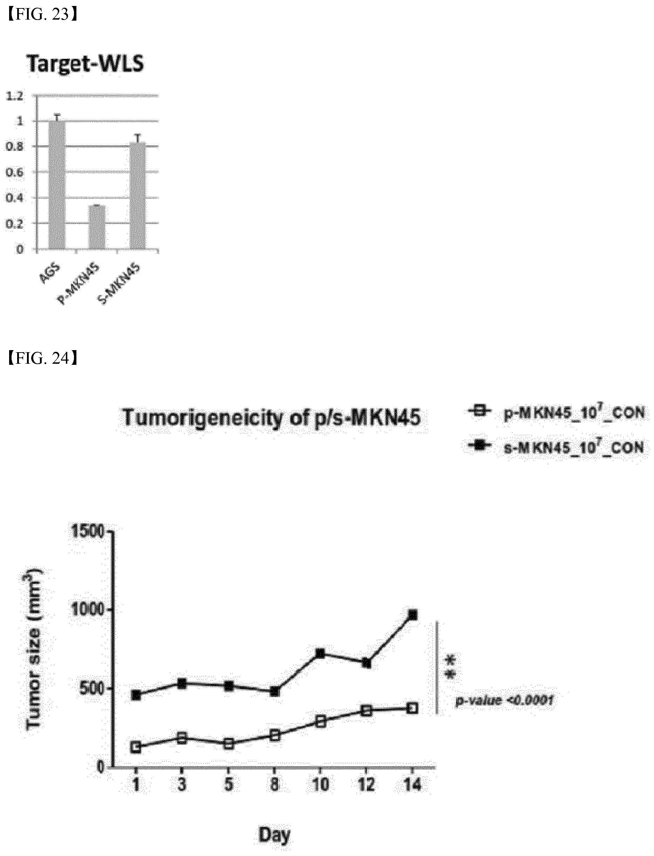

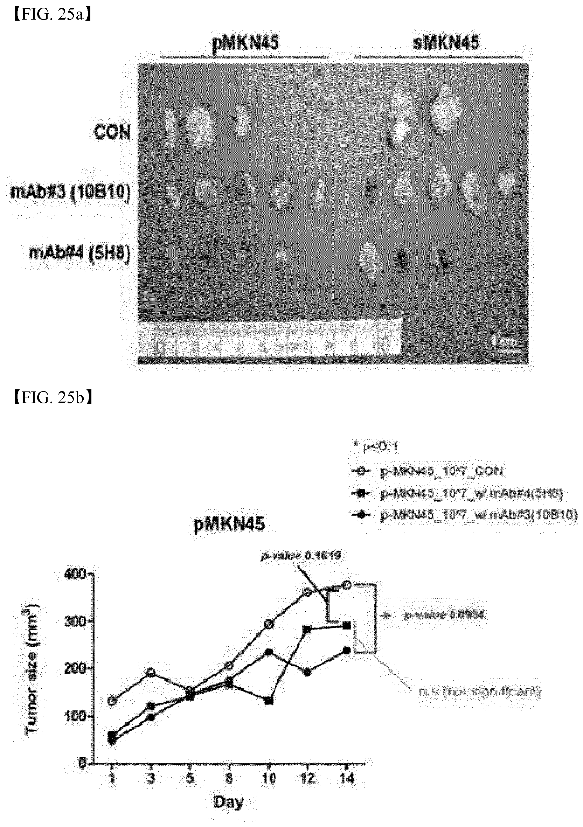

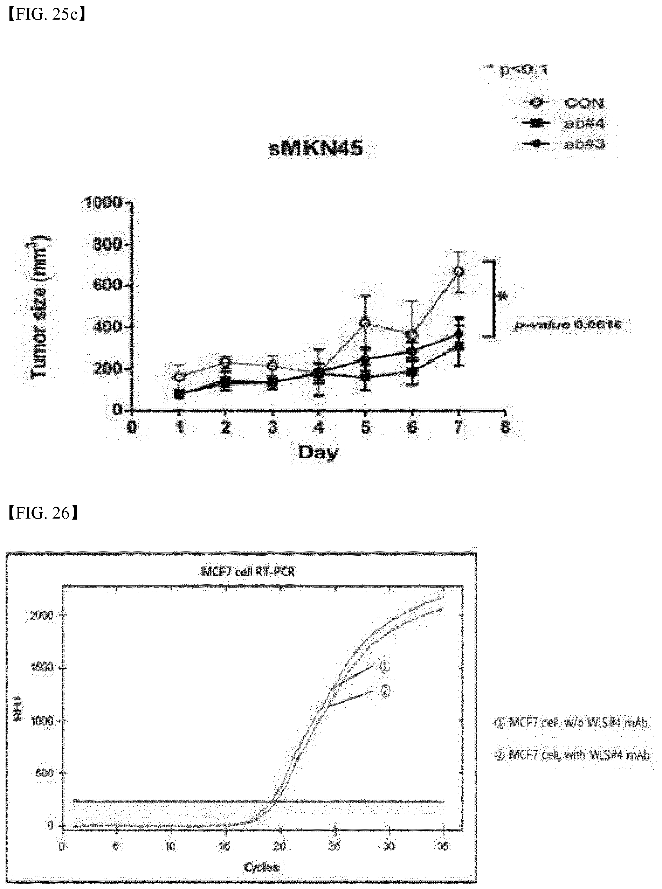

The MKN45 cell line was metabolically starved to establish a selected MKN45 cell line (s-MKN45). The mRNA level of WLS in such s-MKN45 cells was analyzed by a qRT-PCR experiment, and as a result, it was shown that the expression level of WLS in the s-MKN45 cells was higher than that in a non-starved control group (p-MKN45) (FIG. 23). A total of 1 x 10⁷ p-MKN45 cells or s-MKN45 cells mixed with Matrigel at a ratio of 1:2 (v/v) were injected into the subcutaneous tissue of immunodeficient nude mice to form a gastric tumor, and as a result, it was shown that the tumorigenicity of the s-MKN45 cell line was higher than that of the p-MKN45 cell line (FIG. 24). In addition, a total of 4 x 10⁶ p-MKN45 cells or s-MKN45 cells mixed with Matrigel at a ratio of 1:2 (v/v) were injected into the subcutaneous tissue of immunodeficient nude mice to form a gastric tumor, and when the volume of the tumor reached 100 mm³ after about 2 weeks, the monoclonal antibodies constructed in Example 2 were injected through the tail veins. 100 *µ*ℓ of each of the WLS#3 and WLS#4 monoclonal antibodies was injected at a concentration of 1 mg/ml three times for 2 weeks. During the period from the day on which the monoclonal antibody was first injected to two days following the day on which the monoclonal antibody was finally injected, the volume of the volume was measured using calipers according to the following equation: short axis² x long axis/2. As a result, it was shown that the tumor size in both the p-MKN45 cell line and the s-MKN45 cell line in the test group treated with the WLS#3 or WLS#4 monoclonal antibody significantly decreased compared to that in the control group not treated with the antibody (FIGS. 25a to 25c).

### Example 13: Examination of the Change in β-Catenin Expression caused by WLS Monoclonal Antibody

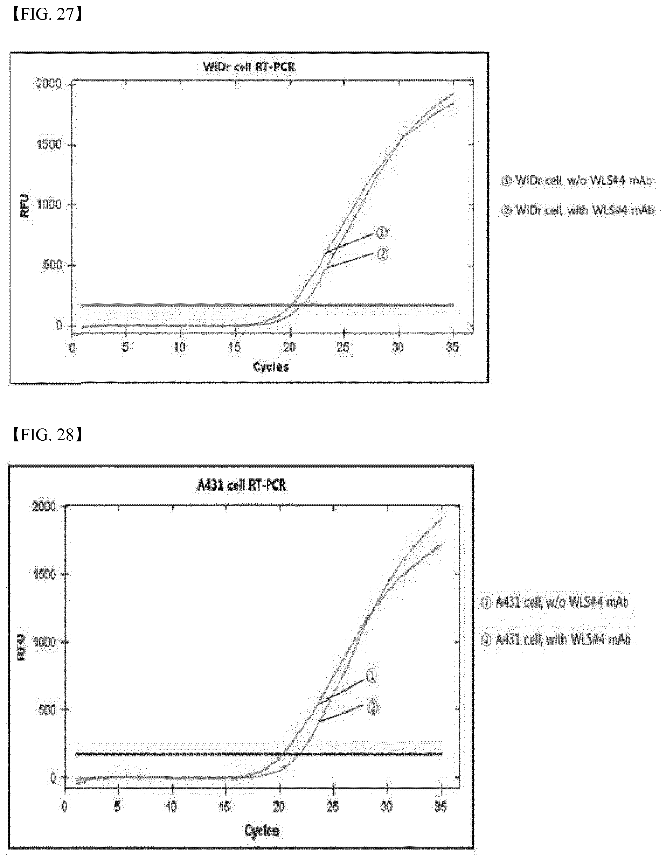

The breast cancer cell line MCF7, the colorectal cancer cell line WiDr and the skin cancer cell line A431 were treated with the WLS#4 monoclonal antibody constructed in Example 2, and after 72 hours, a change in the expression level of β-catenin in each cell line was analyzed by real-time PCR. As a result, it was shown that the expression of β-catenin in all the breast cancer cell line, the colorectal cancer cell line and the skin cancer cell line was reduced by treatment with the WLS#4 monoclonal antibody (FIGS. 26 to 28).

### Example 14: Examination of the Change in β-Catenin Expression caused by WLS Monoclonal Antibody

The breast cancer cell line MCF7, the colorectal cancer cell line WiDr and the kidney cell line 293T were transfected with a Wnt3a-pcDNA3.1 plasmid, and after 24 hours, each of the transfected cell lines was treated for 48 hours with the WLS#4 monoclonal antibody constructed in Example 2. After treatment, a change in the expression level of β-catenin in each cell line was analyzed by real-time PCR.

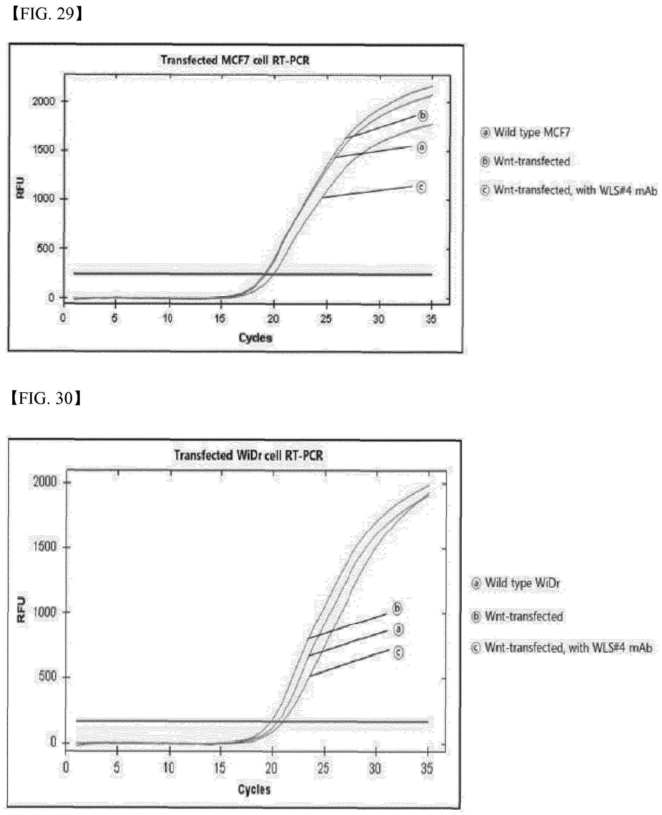

The expression of β-catenin in the breast cancer cell line is as reported in various publications (Carcinogenesis. 2000 Jul;21(7): 1453-6). Although the expression level of β-catenin in the MCF7 cells was also observed to be high, it was shown that the expression level of β-catenin in the MCF7 cells was reduced by treatment with the WLS#4 monoclonal antibody (FIG. 29).

The expression of β-catenin in the colorectal cancer cell line is as reported in various publications (Nature Communications 4, Article number: 2610). Although it could be seen that the expression level of β-catenin in the WiDr cells was also observed to be high and the expression level further increased by continuous stimulation of the Wnt protein, it was shown that the expression level of β-catenin in the WiDr cells was reduced by treatment with the WLS#4 monoclonal antibody (FIG. 30).

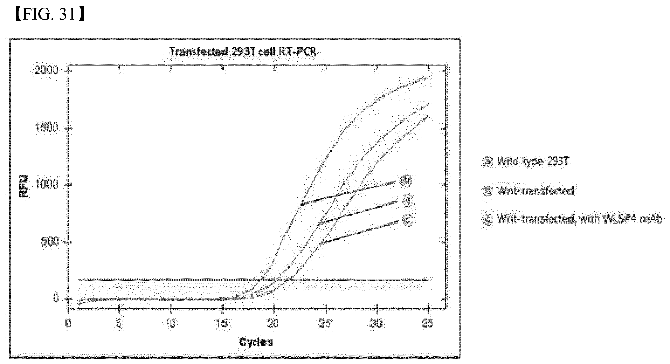

It was reported that abnormalities in the Wnt signaling pathway in kidney tissue cause various diseases, including renal cancer (Organogenesis. 2008 Apr-Jun; 4(2): 55-59). In order to confirm this report, it could be seen that the expression level of β-catenin in the 293T cells was also observed to be high and the expression level further increased by continuous stimulation of the Wnt protein, but it was shown that the expression level of β-catenin in the 293T cells was reduced by treatment with the WLS#4 monoclonal antibody (FIG. 31).

### Example 15: Epitope Mapping for WLS Monoclonal Antibody

Epitope mapping of the peptide (peptide No. 4) for the WLS#4 monoclonal antibody constructed in Example 2 was performed. The amino acid sequence of peptide No. 4 was divided into three amino acid fragments (recombinant peptide Nos. 4-1 to 4-3) as shown in Table 3 below, and each of the fragments was bound to a SNCA-6XHis target to thereby construct recombinant peptides. Using Western blotting, each of the recombinant peptides was reacted with the WLS#4 monoclonal antibody, and the peptide bound to the monoclonal antibody was selected. As a result, it was shown that recombinant peptide No. 4-3 did bind to the WLS#4 monoclonal antibody.

**Table 3**

| Amino acid sequences | Mapping results for peptide No. 4 |
|---|---|
| TFTSPK | Recombinant peptide No. 4-1 |
| TPEHEG | Recombinant peptide No. 4-2 |
| RYYECDV | Recombinant peptide No. 4-3 |

### [Industrial Applicability]

As described above, the present invention is directed to an antibody in preventing or treating a disease associated with the Wnt signaling pathway and to a use of a pharmaceutical composition containing the same.

### [Sequence List Text]

SEQ ID NO. 1: AEWTEMAHERVPRKLKCTFTSPKTPEHEGRYYECDV
SEQ ID NO. 2: AEWTEMAHERVPRKLKCTFT
SEQ ID NO. 3: TFTSPKTPEHEGRYYECDV
SEQ ID NO. 4: TFTSPK
SEQ ID NO. 5: TPEHEG
SEQ ID NO. 6: RYYECDV

<110> ATGEN CO., LTD
<120> An antibody that recognizes a specific motif of WLS protein and a pharmaceutical composition comprising the same
<130> AD 41628 AH/TE
<140> EP 15836263
   <141> 2015-08-26
<150> KR 10-2014-0111398
   <151> 2014-08-26
<160> 12
<170> KoPatentIn 3.0
<210> 1
   <211> 36
   <212> PRT
   <213> homo sapiens
<400> 1
   Ala Glu Trp Thr Glu Met Ala His Glu Arg Val Pro Arg Lys Leu Lys
     1               5                  10                  15
   Cys Thr Phe Thr Ser Pro Lys Thr Pro Glu His Glu Gly Arg Tyr Tyr
                20                  25                  30
   Glu Cys Asp Val
            35
<210> 2
   <211> 20
   <212> PRT
   <213> homo sapiens
<400> 2
   Ala Glu Trp Thr Glu Met Ala His Glu Arg Val Pro Arg Lys Leu Lys
     1               5                  10                  15
   Cys Thr Phe Thr
                20
<210> 3
   <211> 19
   <212> PRT
   <213> homo sapiens
<400> 3
   Thr Phe Thr Ser Pro Lys Thr Pro Glu His Glu Gly Arg Tyr Tyr Glu
     1               5                  10                  15
   Cys Asp Val
<210> 4
   <211> 6
   <212> PRT
   <213> homo sapiens
<400> 4
   Thr Phe Thr Ser Pro Lys
     1               5
<210> 5
   <211> 6
   <212> PRT
   <213> homo sapiens
<400> 5
   Thr Pro Glu His Glu Gly
     1               5
<210> 6
   <211> 7
   <212> PRT
   <213> homo sapiens
<400> 6
   Arg Tyr Tyr Glu Cys Asp Val
   1               5
<210> 7
   <211> 541
   <212> PRT
   <213> homo sapiens
<400> 7
   Met Ala Gly Ala Ile Ile Glu Asn Met Ser Thr Lys Lys Leu Cys Ile
   1               5                   10                  15
   Val Gly Gly Ile Leu Leu Val Phe Gln Ile Ile Ala Phe Leu Val Gly
               20                  25                  30
   Gly Leu Ile Ala Pro Gly Pro Thr Thr Ala Val Ser Tyr Met Ser Val
           35                  40                  45
   Lys Cys Val Asp Ala Arg Lys Asn His His Lys Thr Lys Trp Phe Val
       50                  55                  60
   Pro Trp Gly Pro Asn His Cys Asp Lys Ile Arg Asp Ile Glu Glu Ala
   65                  70                  75                  80
   Ile Pro Arg Glu Ile Glu Ala Asn Asp Ile Val Phe Ser Val His Ile
                   85                  90                  95
   Pro Leu Pro His Met Glu Met Ser Pro Trp Phe Gln Phe Met Leu Phe
               100                 105                 110
   Ile Leu Gln Leu Asp Ile Ala Phe Lys Leu Asn Asn Gln Ile Arg Glu
           115                 120                 125
   Asn Ala Glu Val Ser Met Asp Val Ser Leu Ala Tyr Arg Asp Asp Ala
       130                 135                 140
   Phe Ala Glu Trp Thr Glu Met Ala His Glu Arg Val Pro Arg Lys Leu
   145                 150                 155                 160
   Lys Cys Thr Phe Thr Ser Pro Lys Thr Pro Glu His Glu Gly Arg Tyr
   165 170 175
   Tyr Glu Cys Asp Val Leu Pro Phe Met Glu Ile Gly Ser Val Ala His
   180 185 190
   Lys Phe Tyr Leu Leu Asn Ile Arg Leu Pro Val Asn Glu Lys Lys Lys
   195 200 205
   Ile Asn Val Gly Ile Gly Glu Ile Lys Asp Ile Arg Leu Val Gly Ile
   210 215 220
   His Gln Asn Gly Gly Phe Thr Lys Val Trp Phe Ala Met Lys Thr Phe
   225 230 235 240
   Leu Thr Pro Ser Ile Phe Ile Ile Met Val Trp Tyr Trp Arg Arg Ile
   245 250 255
   Thr Met Met Ser Arg Pro Pro Val Leu Leu Glu Lys Val Ile Phe Ala
   260 265 270
   Leu Gly Ile Ser Met Thr Phe Ile Asn Ile Pro Val Glu Trp Phe Ser
   275 280 285
   Ile Gly Phe Asp Trp Thr Trp Met Leu Leu Phe Gly Asp Ile Arg Gln
   290 295 300
   Gly Ile Phe Tyr Ala Met Leu Leu Ser Phe Trp Ile Ile Phe Cys Gly
   305 310 315 320
   Glu His Met Met Asp Gln His Glu Arg Asn His Ile Ala Gly Tyr Trp
   325 330 335
   Lys Gln Val Gly Pro Ile Ala Val Gly Ser Phe Cys Leu Phe Ile Phe
   340 345 350
   Asp Met Cys Glu Arg Gly Val Gln Leu Thr Asn Pro Phe Tyr Ser Ile
   355 360 365
   Trp Thr Thr Asp Ile Gly Thr Glu Leu Ala Met Ala Phe Ile Ile Val
   370 375 380
   Ala Gly Ile Cys Leu Cys Leu Tyr Phe Leu Phe Leu Cys Phe Met Val
   385 390 395 400
   Phe Gln Val Phe Arg Asn Ile Ser Gly Lys Gln Ser Ser Leu Pro Ala
   405 410 415
   Met Ser Lys Val Arg Arg Leu His Tyr Glu Gly Leu Ile Phe Arg Phe
   420 425 430
   Lys Phe Leu Met Leu Ile Thr Leu Ala Cys Ala Ala Met Thr Val Ile
   435 440 445
   Phe Phe Ile Val Ser Gln Val Thr Glu Gly His Trp Lys Trp Gly Gly
   450 455 460
   Val Thr Val Gln Val Asn Ser Ala Phe Phe Thr Gly Ile Tyr Gly Met
   465 470 475 480
   Trp Asn Leu Tyr Val Phe Ala Leu Met Phe Leu Tyr Ala Pro Ser His
   485 490 495
   Lys Asn Tyr Gly Glu Asp Gln Ser Asn Gly Asp Leu Gly Val His Ser
   500 505 510
   Gly Glu Glu Leu Gln Leu Thr Thr Thr Ile Thr His Val Asp Gly Pro
   515 520 525
   Thr Glu Ile Tyr Lys Leu Thr Arg Lys Glu Ala Gln Glu
   530 535 540
<210> 8
   <211> 20
   <212> PRT
   <213> homo sapiens
<400> 8
   Ile Ala Phe Lys Leu Asn Asn Gln Ile Arg Glu Asn Ala Glu Val Ser
   1 5 10 15
   Met Asp Val Ser
   20
<210> 9
   <211> 20
   <212> PRT
   <213> homo sapiens
<400> 9
   Leu Ala Tyr Arg Asp Asp Ala Phe Ala Glu Trp Thr Glu Met Ala His
   1 5 10 15
   Glu Arg Val Pro
   20
<210> 10
   <211> 20
   <212> PRT
   <213> homo sapiens
<400> 10
   Pro Val Asn Glu Lys Lys Lys Ile Asn Val Gly Ile Gly Glu Ile Lys
   1 5 10 15
   Asp Ile Arg Leu
   20
<210> 11
   <211> 21
   <212> RNA
   <213> Artificial Sequence
<220>
   <223> sense sequence of siRNA siWLS #1
<400> 11
   gucaucuucu ucaucguuau u 21
<210> 12
   <211> 21
   <212> RNA
   <213> Artificial Sequence
<220>
   <223> antisense sequence of siRNA siWLS #1
<400> 12
   uaacgaugaa gaagaugacu u 21

## Claims

1. A composition for use in a method of preventing or treating cancer associated with a Wnt signaling pathway, the composition containing an antibody specific for a protein represented by the amino acid sequence of SEQ ID NO: 2.

2. The composition for use of claim 1, wherein the cancer is selected from the group consisting of small-cell lung cancer, non-small-cell lung cancer, breast cancer, prostate cancer, carcinoid, bladder cancer, gastric cancer, pancreatic cancer, liver cancer, colon cancer, rectal cancer, colorectal cancer, renal cancer, head cancer, bone cancer, neck squamous cell carcinoma, esophageal cancer, ovarian cancer, cervical cancer, endometrial cancer, mesothelioma, melanoma, sarcoma, osteosarcoma, liposarcoma, thyroid cancer, desmoids, acute myelocytic leukemia (AML), and chronic myelocytic leukemia (CML).

3. A composition for use in a method of diagnosing cancer or cancer metastasis, the composition containing an antibody specific for a protein represented by the amino acid sequence of SEQ ID NO: 2.

4. The composition for use of claim 3, wherein the cancer is selected from the group consisting of small-cell lung cancer, non-small-cell lung cancer, breast cancer, prostate cancer, carcinoid, bladder cancer, gastric cancer, pancreatic cancer, liver cancer, colon cancer, rectal cancer, colorectal cancer, renal cancer, head cancer, bone cancer, neck squamous cell carcinoma, esophageal cancer, ovarian cancer, cervical cancer, endometrial cancer, mesothelioma, melanoma, sarcoma, osteosarcoma, liposarcoma, thyroid cancer, desmoids, acute myelocytic leukemia (AML), and chronic myelocytic leukemia (CML).

5. A method for screening a cancer therapeutic agent, the method comprising determining that a candidate substance for cancer treatment is a co-agent for cancer therapy when expression of WLS protein is inhibited in a case where an antibody specific for a protein represented by SEQ ID NO: 2 is administered *in vitro* in combination with the candidate substance, compared to a case where the antibody for the WLS motif protein is administered alone.

## Patentansprüche

1. Zusammensetzung zur Anwendung in einem Verfahren zur Vorbeugung oder Behandlung von mit einem Wnt-Signalweg in Verbindung stehendem Krebs, wobei die Zusammensetzung einen Antikörper enthält, der für ein Protein spezifisch ist, das durch die Aminosäuresequenz der SEQ ID NO: 2 repräsentiert wird.

2. Zusammensetzung zur Anwendung nach Anspruch 1, wobei der Krebs ausgewählt ist aus der Gruppe, die aus kleinzelligem Lungenkrebs, nicht-kleinzelligem Lungenkrebs, Brustkrebs, Prostatakrebs, Karzinoid, Blasenkrebs, Magenkrebs, Bauchspeicheldrüsenkrebs, Leberkrebs, Darmkrebs, Rektumkrebs, Darmkrebs, Nierenkrebs, Kopfkrebs, Knochenkrebs, Plattenepithelkarzinom des Halses, Speiseröhrenkrebs, Eierstockkrebs, Gebärmutterhalskrebs, Endometriumkrebs, Mesotheliom, Melanom, Sarkom, Osteosarkom, Liposarkom, Schilddrüsenkrebs, Desmoiden, akuter myelozytischer Leukämie (AML) und chronischer myelozytischer Leukämie (CML) besteht.

3. Zusammensetzung zur Anwendung in einem Verfahren zur Diagnose von Krebs oder Krebsmetastasen, wobei die Zusammensetzung einen Antikörper enthält, der für ein Protein spezifisch ist, das durch die Aminosäuresequenz der SEQ ID NO: 2 repräsentiert wird.

4. Zusammensetzung zur Anwendung nach Anspruch 3, wobei der Krebs ausgewählt ist aus der Gruppe, die aus kleinzelligem Lungenkrebs, nicht-kleinzelligem Lungenkrebs, Brustkrebs, Prostatakrebs, Karzinoid, Blasenkrebs, Magenkrebs, Bauchspeicheldrüsenkrebs, Leberkrebs, Darmkrebs, Rektumkrebs, Darmkrebs, Nierenkrebs, Kopfkrebs, Knochenkrebs, Plattenepithelkarzinom des Halses, Speiseröhrenkrebs, Eierstockkrebs, Gebärmutterhalskrebs, Endometriumkrebs, Mesotheliom, Melanom, Sarkom, Osteosarkom, Liposarkom, Schilddrüsenkrebs, Desmoiden, akuter myelozytischer Leukämie (AML) und chronischer myelozytischer Leukämie (CML) besteht.

5. Verfahren zum Durchmustern eines Krebstherapeutikums, wobei das Verfahren aufweist, zu bestimmen, dass eine Kandidatensubstanz für die Krebsbehandlung ein Co-Wirkstoff für die Krebstherapie ist, wenn im Fall, dass bei *in vitro*-Verabreichen der Kandidatensubstanz in Kombination mit ein Antikörper, der für ein Protein spezifisch ist, das durch die Aminosäuresequenz der SEQ ID NO: 2 repräsentiert wird, die Expression des WLS-Proteins inhibiert wird, verglichen mit dem Fall, dass der Antikörper für das WLS-Motivprotein allein verabreicht wird.

## Revendications

1. Composition à utiliser dans un procédé de prévention ou de traitement d'un cancer associé à une voie de signalisation Wnt, la composition contenant un anticorps spécifique à une protéine représentée par la séquence d'acides aminés de SEQ ID n° 2.

2. Composition à utiliser selon la revendication 1, dans lequel le cancer est sélectionné dans le groupe constitué par le cancer du poumon à petites cellules, le cancer du poumon non à petites cellules, le cancer du sein, le cancer de la prostate, un carcinoïde, le cancer de la vessie, un cancer gastrique, un cancer pancréatique, le cancer du foie, le cancer du côlon, un cancer rectal, un cancer colorectal, un cancer rénal, le cancer de la tête, le cancer des os, un carcinome des cellules squameuses du cou, un cancer œsophagien, un cancer ovarien, le cancer du col utérin, le cancer de l'endomètre, un mésothéliome, un mélanome, un sarcome, un ostéosarcome, un liposarcome, le cancer de la thyroïde, des tumeurs desmoïdes, la leucémie myélocytaire aiguë (LMA) et la leucémie myélocytaire chronique (LMC).

3. Composition à utiliser dans un procédé de diagnostic d'un cancer ou d'une métastase de cancer, la composition contenant un anticorps spécifique à une protéine représentée par la séquence d'acides aminés de SEQ ID n° 2.

4. Composition à utiliser selon la revendication 3, dans laquelle le cancer est sélectionné à partir du groupe constitué par le cancer du poumon à petites cellules, le cancer du poumon non à petites cellules, le cancer du sein, le cancer de la prostate, un carcinoïde, le cancer de la vessie, un cancer gastrique, un cancer pancréatique, le cancer du foie, le cancer du côlon, un cancer rectal, un cancer colorectal, un cancer rénal, le cancer de la tête, le cancer des os, un carcinome des cellules squameuses du cou, un cancer œsophagien, un cancer ovarien, le cancer du col utérin, le cancer de l'endomètre, un mésothéliome, un mélanome, un sarcome, un ostéosarcome, un liposarcome, le cancer de la thyroïde, des tumeurs desmoïdes, la leucémie myélocytaire aiguë (LMA) et la leucémie myélocytaire chronique (LMC).

5. Procédé pour cribler un agent thérapeutique de cancer, le procédé comprenant la détermination qu'une substance candidate pour le traitement d'un cancer est un co-agent pour la thérapie d'un cancer quand l'expression de la protéine WLS est inhibée dans un cas où un anticorps spécifique à une protéine représentée par SEQ ID n° 2 est administré *in vitro* en combinaison avec la substance candidate, en comparaison à un cas où l'anticorps pour la protéine de motif WLS est administré seul.
